**Europäisches Patentamt**

(19) **European Patent Office**

**Office européen des brevets**

(11) Publication number : **0 410 412 B1**

# (12) EUROPEAN PATENT SPECIFICATION

(45) Date of publication of patent specification : **05.01.94 Bulletin 94/01**

(51) Int. Cl.$^5$ : **C09J 193/04,** C09J 11/06, // (C09J193/04, 123:08, 153:02)

(21) Application number : **90114251.3**

(22) Date of filing : **25.07.90**

(54) **Hot melt adhesive having controlled property change.**

(30) Priority : **25.07.89 US 385315**

(43) Date of publication of application : **30.01.91 Bulletin 91/05**

(45) Publication of the grant of the patent : **05.01.94 Bulletin 94/01**

(84) Designated Contracting States : **AT BE CH DE DK ES FR GB GR IT LI LU NL SE**

(56) References cited :
**EP-A- 0 104 005**
**EP-A- 0 340 990**
**WO-A-90/00065**
**WO-A-90/01525**
**GB-A- 911 821**
**US-A- 2 454 676**
**US-A- 4 745 026**

(73) Proprietor : **H.B. FULLER LICENSING & FINANCING, INC.**
**1100 North Market Street, Suite 780**
**Wilmington, Delaware 19801 (US)**

(72) Inventor : **Bunnelle, William L.**
**10151 180th Street North**
**Hugo Township, MN 55038 (US)**
Inventor : **Ryan, Christopher M.**
**1130 256th Street**
**Chisago City, MN 55013 (US)**

(74) Representative : **Strasse, Maiwald, Meys, Stach & Vonnemann**
**Postfach 90 09 54**
**D-81509 München (DE)**

## Description

The invention relates to a novel class of hot melt adhesives that can exhibit a controlled property change when cooled after application. The adhesives can be used to form high quality permanent bonds or can be used to form "fugitive bonds." Permanent bonds can exhibit highly cohesive, tensile, peel and shear strength sufficient to maintain the mechanical integrity of a structure manufactured with the adhesive. Such adhesives can be used in a variety of construction applications such as in composite articles or on hard to stick surfaces such as fluorocarbons or high clay filled surfaces. A fugitive bond is formed by an adhesive that is formulated to form an initially strong cohesive bond under peel and shear but, after time, changes to a brittle bond that has significant shear strength but has little peel strength. Such adhesives can be used in a variety of end uses including easily opened case and carton sealing adhesives, can and bottle labeling adhesives, palletizing adhesives, etc.

Background of the Invention

Thermoplastic hot melt adhesives of the prior art typically comprise a thermoplastic polymer, a tackifier, a wax or plasticizing oil, and other optional ingredients. In large part, hot melt adhesive development in recent years has focused on the thermoplastic polymer used as the adhesive base material. Many inventions are based on finding a new property in a new thermoplastic polymer/adhesive composition or finding a new use for a known adhesive. A large variety of literature is directed to various thermoplastic polymers such as ethylene/vinyl acetate, ethylene methacrylate, atactic polypropylene, A-B-A block copolymers, $A(BA)_nB$ block copolymers, etc. A brief selection of such literature includes:

Skeist, Handbook of Adhesives, Van Nostrand Reinhold Co. Inc. (1977).

Battersby et al., U.S. Pat. No. 3,318,977, teaches thermoplastic adhesives containing polyethylene, isobutylene rubber, tackifier resins, and ethylene-vinyl acetate copolymers.

Meeks et al. , U.S. Pat. No. 3,971,883, teaches crosslinkable ethylene vinyl acetate copolymer resins in adherent laminates.

Taft et al., U.S. Pat. No. 3,982,051, teaches hot melt adhesive compositions containing ethylene/vinyl acetate and/or alkyl acrylate copolymers in hot melt carpet backing adhesives.

Boggs, U.S. Pat. No. 4,299,930, teaches hot melt adhesives containing modified polyethylenes and ethylene vinyl acetate copolymer and other materials.

Eastman, U.S. Pat. No. 4,293,473, teaches polyvinyl alcohol or ethylene vinyl alcohol copolymers in crystalline solvent based systems for bonding cellulosics, spun bonded polyolefins, aluminum foil, and other substrates.

Flanagan et al., U.S. Pat. No. 4,345,349, teaches a combination of an A-B-A block copolymer and ethylene vinyl acetate polymer and other standard hot melt ingredients to form an adhesive for book binding.

Tancrede et al., U.S. Pat. No. 4,497,936, teaches ethylene-vinyl acetate copolymers in combination with olefin rubbers in hot melt adhesives.

The adhesives disclosed in the typical prior art publications are adhesives that are applied as a melt liquid, and when cooled to ambient temperature become a solid. Once cooled, the adhesive mass attains relatively stable properties such as peel strength, shear strength, cold flow, or storage modulus, also known as G'. In other words, upon cooling, the adhesive formulation rapidly reaches a near equilibrium condition with respect to its physical state. As a result, each physical property rapidly attains the vast majority of its values immediately when cooled. We will refer to this state immediately after a hot melt adhesive cools and attains near equilibrium properties as the "ambient state" of the hot melt adhesive.

In the conventional production of prior art work pieces or articles, conventional hot melt adhesives are typically extruded at elevated temperature directly onto a bond site. The second substrate must be combined with the adhesive relatively quickly while the adhesive is still molten or liquid, or the adhesive will cool, harden, and set, making it impossible for the melt to wet the second substrate to form a bond. When the molten adhesive is extruded in a form of hot molten bead onto a porous substrate, the adhesive can wet or soak into one or more porous substrate and adhere the substrate to another substrate by entrapping fibers in the cooled adhesive mass. In such hot melt technology, the molten adhesive retains sufficient heat such that the material can retain a low melt viscosity, low modulus, and can penetrate or wet components of a work piece to ensure secure bonding before cooling. As such adhesives cool, the properties rapidly reach a final ambient state and, once the final values are reached, have, for the most part, stable values for that adhesive blend at ambient temperatures. However, such adhesives have the drawback that bonds must be made while the adhesive is at an elevated temperature to insure proper bonding, which prevents their use on heat sensitive substrates.

Pressure sensitive adhesive have been developed based on thermoplastic elastomers and a large variety of patents are directed to elastomeric based adhesives. A brief selection of such patents include:

Collins et al, U.S. Patent No. 4,136,699, teaches a disposable article using a hot melt pressure-sensitive adhesive as a positioning or a construction material. Such adhesive is typically extruded at high temperature onto materials of construction during manufacture.

Chen et al, U.S. Patent No. 4,460,364, teaches hot melt pressure-sensitive adhesives used in the manufacture of sanitary products.

Schmidt Jr., et al, U.S. Patent No. 4,525,577, teaches the use of styrene-butadiene-styrene block copolymers in the manufacture of disposable laminates using multi-line extrusion adhesive technology.

Puletti et al, U.S. Patent No. 4,627,847, also teaches the use of hot melt adhesives and disposable article construction.

Elastomeric based products have been developed which exhibit the property of pressure sensitivity. These types of products can form a bond after cooling, but generally do not cold flow after the adhesive reaches the ambient state. These elastomeric based pressure sensitive products are unable to form mechanical bonds or physically entrap fibers of a porous substrate after they are cooled and reach their ambient state. They tend to form surface bonds from the pressure sensitive nature of the adhesive.

Adhesives having properties that vary after application and cooling would offer significant advantages. In certain applications, initial bond strength is important, while after a time, the bond strength is preferably reduced. In other applications, fluidity is important while, after a time, the compositions are preferably in solid form. In still other applications, initial bond strength is not critical, while a high final bond strength is critical.

Accordingly, a substantial need exists in the industry for an adhesive that can have a controlled change in important properties after application. In other words, a need exists for an adhesive that does not reach a final equilibrium value for one or more physical properties until well after application and cooling.

In one aspect, a controlled change in modulus can provide important advantages. After application and cooling, the modulus of the adhesive is as an intermediate state between the modulus of the molten mass and the final modulus and is significantly below the modulus of typical comparable prior art hot melt adhesives. Such an adhesive with a controlled change or increase in modulus (G') can initially obtain significant cold flow after application that aids in construction of a variety of work pieces. Such cold flow can cause the adhesive to flow into a fabric to cause the physical entrapment of the materials of construction in the adhesive mass resulting in the formation of a bond of high integrity when the adhesive reaches its modulus potential. Such cold flow can also cause rapid and enhanced surface wetting of a nonporous surface producing enhanced bonding when fiber entrapment is not involved. Further, the bond can significantly resist the effects of a number of debonding mechanisms, including the presence of moisture or other compositions that can reduce or eliminate bond strength. After a time, the modulus increases to a final high equilibrium value forming strong cohesive bonds.

In recent years, increasing attention has been directed to the development of hot melt adhesives that can be sprayed onto the work piece or substrate during a manufacturing regimen. The use of spray-on adhesives has been found to increase productivity. Conventional spray-on adhesives are sprayed from a plurality of narrow orifices in a form of a fiber, a thread, a filament, or a plurality thereof, having a substantially circular cross-section with a diameter of 0.025 cm (0.01") to 0.102 cm (0.04"). The spray-on adhesive fiber has substantial surface area in comparison to the mass or volume of the fiber. As a result, the sprayed adhesive fiber cools very rapidly upon contact with the ambient atmosphere. However, the spray-on adhesive, even if it retains some residual heat, attains the ambient temperature very quickly upon contact with the work piece. By ambient temperature, we mean the temperature of the surrounding atmosphere and the temperature of the work piece in the construction locale. This is in sharp contrast to extruded hot melt adhesives that retain significant amounts of heat for a period after application. Most conventional spray-on adhesives require a heated air flow at a temperature exceeding 121°C (250°F)., which is above the $T_g$ of styrene in the block copolymers used in the adhesive. Such a temperature is required to keep the adhesive molten until applied. In these spray-on adhesive applications, the temperature of the work piece and the manufacturing locus (not including the application equipment) are typically not substantially different. Conventional spray-on adhesives, after their application on work pieces, typically form a solid mesh or a web which is the result of the pattern in which the spray-on adhesive is applied to the substrate. An overlapping application pattern, as it is directed onto a moving web, typically takes the form of overlapping circles or ovals of adhesives that form a continuous adhesive strip or layer.

Our copending patent application WO/00065 (EP) 89 908 052.7), not published at the priority date of the instant application, teaches sprayable hot melt adhesives having superior wet bond strength. These adhesive compositions comprise thermoplastic block copolymers, namely A-B-A block copolymers, A-(B-A)$_n$-B-A block copolymers (n $\geq$ 1) and radial block copolymers (A being a polystyrene block and B a rubbery block) besides rosin tackifying resin and a plasticizer, which has a softening point of above about 45°C. These compositions

EP 0 410 412 B1

are suitable for adhering a moisture-absorbent material to a substrate, e.g. bonding the cellulosic nonwoven of a disposable diaper or feminine pad to the back sheet, overcoming the delamination or debonding problems encountered when using prior art hot melt adhesive compositions in such applications.

In another aspect, an adhesive with controlled bond strength can be important in a pallitizing adhesive and a carton-sealing adhesive which can have easily opened bonding. In such applications, initial peel and shear strength are important to secure the components in place. After assembly, the adhesive bond needs only sufficient shear strength to maintain the integrity of the assembly. At a use locus, the carton adhesive and the palletizing adhesive preferably have low peel strength permitting easy opening of the carton or easy disassembly of the pallet.

GB-B-911 821 discloses adhesives consisting mainly of a rosin or modified rosin and a plasticizer, which can be dicyclohexyl phthalate. These prior art adhesives are no hot melt adhesives, but emulsions or solutions in aqueous systems. They are intended for adhering films to e.g. labels through delayed heat-seal action.

US-B-2 454 676 discloses an adhesive of very limited cohesion (failure typically at less then 35 g/cm$^2$ of vertical shear stress in less than 30 min.). These prior art adhesives are coextrudates of rosins and plasticizers such as triethylene glycol-di-2-ethyl-butyrate. They are also no hot melts, but oily pastes (at ambient temperatures) and are used at room temperature without being heated.

None of these adhesives exhibits a controlled change of physical properties after application.

Brief Discussion of the Invention

We have found a novel class of adhesive compositions containing proportions of a tackifier and a plasticizer that are selected by using selective compatibility to produce, in the adhesive, properties that vary after application. The adhesive, when applied and cooled, exhibits one or more physical properties that do not attain a final or equilibrium value (ambient state) until at least 5 minutes after application and cooling. By final or equilibrium value, we mean the value of a physical property after the vast majority of changes in the property and the vast majority of changes in the adhesive mass have occurred.

The adhesives of the invention can have any arbitrary amount of thermoplastic polymer. At a typical polymer content, the adhesive, when first applied, has low modulus, while the final bonds obtain significantly high equilibrium modulus, peel and shear strength. At no polymer to low relative polymer concentration, the initial bonds have sufficient tack, peel strength and shear strength to hold the substrates together and act as case or carton sealing adhesives, but at equilibrium tend to have poor peel strength but have sufficient modulus for many label pickup or case and carton adhesive applications. The low peel strength and brittle nature of these low polymer adhesives result in a palletizing adhesive of easy release from the pallet and easy opening case or carton adhesive, otherwise known as a fugitive bond.

A controlled increase in modulus or G' after the adhesive has been applied and cooled can be obtained. The increase in modulus is at least ten times the modulus (G') of the adhesive immediately after adhesive application and cooling. Further, we have found that novel adhesive compositions of this invention can be prepared from a tackifier-plasticizer composition, wherein the increase in modulus can be controlled by variation in the compatibility of plasticizer/tackifier combination and their amount, such that the modulus reaches steady state at a specific desired period after application. In other words, the maximum modulus can be attained within a few minutes after application, within about 60 minutes of application, or more than 4 hours of application, as desired. However, it is clear that the adhesives of this invention typically do not attain a final modulus until at least 5 to 10 minutes after adhesive application to the work piece and cooling. The adhesives of the invention can be applied without the constraints resulting from the nature of the conventional hot melts. The adhesives of the invention can be applied at an adhesive temperature below 121°C (250°F)., preferably below 66°C (150°F)., and, most preferably, below 52°C (125°F).

A hot melt adhesive with suitable "creep" or "cold flow" properties will have some tendency to flow in the temperature range of 25°-50°C., but this tendency should be controlled, as manifested by a controlled change in modulus (G') values.

The following are considered to be illustrative values of storage modulus (G') values. The values are to be measured and recorded just after application and cooling (application modulus), and after the modulus has increased to a final equilibrium value (high steady state modulus).

| Property | Values at 25-50°C. and 0.01-0.25 Hz |
|---|---|
| Application Modulus (G') | 0.4 to 5 N/cm² |
| High Steady State Modulus (G') | 4 to 350 N/cm² |

Test Method for Controlled Increasing Modulus Samples Scope

This method has been developed to demonstrate and measure the phenomena of delayed and controlled modulus increase in samples of hot melt adhesive.

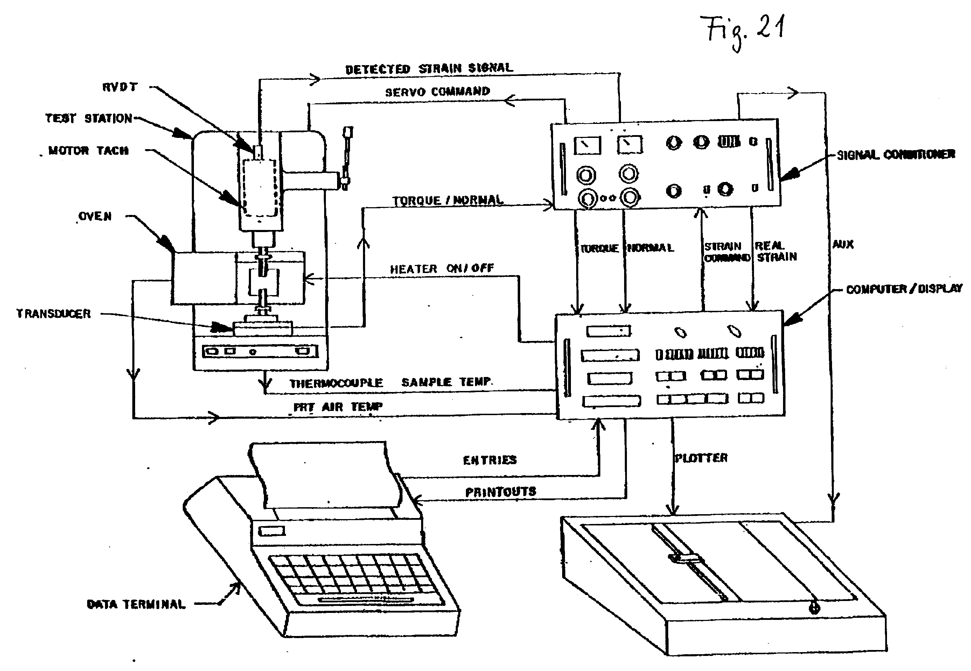

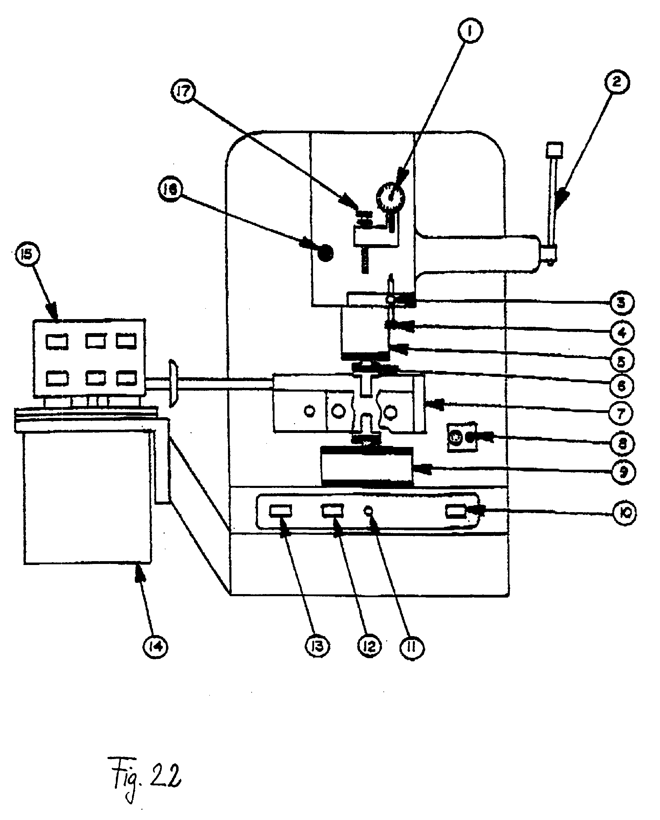

Fig. 21 shows a diagram of a rheological dynamic spectrometer while Fig. 22 shows the test station of said spectrometer in greater detail.

In Fig. 22, the reference numerals indicate:

| | Control/Indicator | Function |
|---|---|---|
| 1 | GAP INDICATOR | Scaled in increments of .001mm; this meter shows precise gap between the platens. |
| 2 | SPINDLE ADJUST | Lowers the spindle when pulled forward. Used to raise the spindle during tool and sample insertion. |
| 3 | COARSE INDICATOR ADJUST | Used to move vertical slide so Gap Indicator reads approximately zero with platens in contact. |
| 4 | FINE INDICATOR ADJUST | A fine adjustment of Gap Indicator zero with the platens touching. |
| 5 | SPINDLE | Houses the servo motor for application of strain to the sample. |
| 6 | TOOL LOCKING RINGS | Fixtures for different testing modes are precision fit and secured by the tool locking rings. Removal directions are engraved in the upper spindle and motor cover plate. |
| 7 | ENVIRONMENTAL CHAMBER | Allows temperature control of the sample under test. Several types of Chambers are used depending on the test fixture selected. |
| 8 | OVEN LIGHT | Switch and outlet for lamp in Environmental Chamber. When pressed, the interior of the Environmental Chamber is illuminated. |
| 9 | TRANSDUCER | Detects the Torque and Normal Force applied to the sample under test and supplies the Computer with the resultant information. |

| PANEL CONTROLS: | | |
|---|---|---|
| 10 | ENVIRONMENTAL CHAMBER | When pressed, enables the Environmental Chamber to raise or lower the temperature of the sample to the selected temperature. |
| 11 | HEATER Indicator | Lights when the heater is on. Flashing indicates the desired temperature has been met and stabilization is being maintained. |
| 12 | MOTOR | Applies or removes power to the drive motor and places the motor under Computer control. |
| 13 | MAIN POWER | Applies or removes power to the Rheometrics Dynamic Spectrometer. Applying power to the system causes the Data Terminal to print the current RDS SOFTWARE VERSION. |
| 14 | DEWAR | A vessel with evacuated walls for holding liquid nitrogen. Allows temperature control of the Environmental Chamber to $-150^\circ$C. |
| 15 | LIQUID NITROGEN CONTROLLER | Controls the level to which liquid nitrogen is allowed to fill. Also controls application of power to the $N_2$ Immersion Heater. |
| 16 | SPINDLE LOCK | Clockwise rotation locks the spindle at any position in the spindles travel. |
| 17 | POSITION STOP | A mechanical stop adjusted to where the upper and lower platens just touch as indicated by the Normal Force panel meter. |

Materials and Equipment

1. Rheometric rheological dynamic spectrometer/model TC-2000
2. 1.25 cm radius disposable plates

3. Release paper or Teflon board

The term storage modulus (G') and other related viscoelastic or rheological properties, such as loss tangent (tan delta), loss modulus (G"), and loss compliance (J'), are interrelated and are defined by established methods of dynamic mechanics. These rheological quantities are measured on samples approximately 2.5 mm in thickness placed between 25 cm parallel plate fixtures of a Rheometrics Rheological Spectrometer Model TC-2000.

Procedure for First Run of Samples

1. Melt sample in oven at 177°C (350°F) for 20-30 minutes.
2. Pour a puddle of adhesive onto release paper; try to avoid making bubbles.
3. Find a bubble-free portion of the sample and place it on the disposable plate of the RDS. Cut off the excess adhesive with either a razor blade or a scissors. Mark the plate with the sample ID and mark its bottom.
4. Place the sample in the bottom sample holder and tighten the set screw.
5. Place another disposable plate in the upper fixture and tighten the set screw.
6. Mark both plates to show where they were aligned in the fixture by marking a line on the plate directly under the slit on the RDS fixture.
7. Bring the armature down so that the upper plate just makes contact with the sample. Watch the normal force and make enough contact to just barely cause deflection on the needle.
8. Melt the sample by raising the temperature to 90-100°C. This will vary depending on the softening point of the sample. Watch the sample and soften it as much as you dare so as not to cause it to drip out of the fixture.
9. Allow it to equilibrate for 10 minutes.
10. Start the time sweep by pressing the START button.
11. Immediately adjust the temperature setting to 25°C.
12. Watch the RDS to make sure that it gives readings for the time and temperature intervals. Also, be sure that the normal force scale remains between the green portion on the scale for optimal readings. Be sure that the normal force does not exceed 25%. Forces greater than this could damage the transducer.
13. When the run is complete remove the sample by loosening the set screw for the upper fixture first. Be sure that the normal force is within 25% when lifting the fixture off of the sample plate. Again, forces greater than 25% may damage the transducer.
14. Mark the sample for the time of the run on the top plate.

RDS Settings

Initial Settings (Day 1)

Purge Temperature

Air + 0.276 N/mm² (40 psi)
(coil in ice bath)
Equilibrate at 90-110°C for 10 minutes depending on the sample. Then start the time sweep and immediately set the temperature to 25°C.

Rate

1 rad/sec

Strain

1%

Sweep Parameters

Total Time:  90 min.

Time/Measurement:  60 min.

Test Geometry

Gap:    2-4 mm

Radius: 1.25 cm

Print

G'

G"

Tandelta

Temperature

Torque

Time

Procedure for Subsequent Runs of Samples

1. Place the sample in the lower test fixture, making sure to line up the alignment mark on the plate with the lines in the fixture. Tighten the set screw.
2. Bring down the upper fixture and connect the sample in the holder. Make sure to watch so that the normal force does not exceed 25% so not to cause damage to the transducer. Tighten the set screw.
3. Set the gap width to the same setting use for measurement on DAY 1.
4. Make appropriate settings for a 30-minute time sweep at constant (25°C.) temperature.

Settings for Day 1

Purge               Air + 0.276 N/mm² (40 psi) (coil in ice bath)

Temperature    · ·   25°C.         constant

Sweep Parameters    Total Time:         30 min.

                    Time/Measurement:  ⌐ 3 min.

Test Geometry       Gap:    Do NOT measure and reset the

                            reading, use the reading

                            found for the initial

                            setting.

                    Radius: 1.25 cm

Detailed Description of the Invention

The adhesive typically comprises a tackifying resin, which is typically aliphatic, aromatic, or aliphatic-aromatic in character, a plasticizer solid at room temperature, and, optionally, an effective amount of thermoplastic polymer and an optional oil. The components are selected in proportions such that the adhesive exhibits varied properties as described above. The rate of property change can be controlled by selection of appropriate tackifying resin, or blend of resin, and plasticizer in proportions of tackifiers and plasticizer such that the desired modulus can reach its maximum value in a controlled period, i.e., from greater than about 10 minutes, 1 hour, 6 hours or more, after application.

We have found that these components cooperate to form an adhesive having improved properties. The adhesives can have low viscosity at application temperatures. The adhesive has extended open time, useful viscosity profile, and excellent wet and dry bond strength.

The typical compositions of these adhesives are set forth below in Tables I and II (the numerical values including weight percent content in the overall composition).

TABLE I

Low Polymer Resin Compositions

|  | Useful | Preferred | Most Preferred |
|---|---|---|---|
| Solid Plasticizer | 5-50 | 10-50 | 40-50 |
| Tackifier | 20-80 | 30-80 | 35-65 |
| Polymer | 0-15 | 0-12 | 0.1-10 |

EP 0 410 412 B1

## TABLE II

### Higher Polymer Resin Compositions

|  | Useful | Preferred | Most Preferred |
|---|---|---|---|
| Solid Plasticizer | 5-50 | 10-50 | 20-30 |
| Tackifier | 20-80 | 30-75 | 35-60 |
| Polymer | 10-55 | 12-35 | 15-30 |

Tackifying Resin

The adhesives of the invention may contain a tackifying resin in combination with a thermoplastic polymer and the plasticizer. Tackifying resins useful in the adhesives of the invention comprise rosin derivatives including wood rosin, tall oil, tall oil derivatives, rosin ester resins, natural and synthetic terpenes and aromatic, aliphatic or mixed aromatic-aliphatic tackifying resins. The tackifying resins are selected for a specific degree of compatibility with the plasticizer.

Aromatic monomers useful in forming the aromatic and aliphatic-aromatic adhesive compositions of the invention can be prepared from any monomer containing substantial aromatic qualities and a polymerizable unsaturated group. Typical examples of such aromatic monomers include the styrenic monomers styrene, alphamethylstyrene, vinyl toluene, methoxystyrene, t-butylstyrene, chlorostyrene, etc., indene monomers including indene, methyl indene, and others. Aliphatic monomers are typically natural and synthetic terpenes which contain $C_5$ and $C_6$ cyclohexyl or cyclopentyl saturated groups that can additionally contain a variety of substantially aliphatic ring substituents. Aliphatic tackifying resins can be made by polymerizing a feed stream containing sufficient aliphatic monomer such that the resulting resin exhibits aliphatic characteristics. Such feed streams can contain other aliphatic unsaturated monomers such as 1,3-butadiene, cis-1,3-pentadiene, trans-1,3-pentadiene, 2-methyl-1,3-butadiene, 2-methyl-2-butene, cyclopentadiene, dicyclopentadiene, terpene monomers, and others. Mixed aliphatic-aromatic resins contain sufficient aromatic monomers and sufficient aliphatic monomers and optionally other $C_3$-$C_8$ unsaturated monomers to produce a resin having both aliphatic and aromatic character.

The adhesive compositions of the invention can contain rosin and rosin derivatives as a tackifying agent. Rosin is a solid material that occurs naturally in the oleo resin of pine trees and typically is derived from the oleo resinous exudate of the living tree, from aged stumps and from tall oil produced as a by-product of kraft paper manufacture. After it is obtained, rosin can be treated by hydrogenation, dehydrogenation, polymerization, esterification, and others. Rosin is typically classed as a gum rosin, a wood rosin, and as a tall oil rosin. The materials can be used unmodified and additionally can be used in the form of esters of polyhydric alcohols and can be polymerized through the inherent unsaturation of the molecules. The materials are commercially available and can be blended into the adhesive compositions using standard blending techniques.

Representative examples of such rosin derivative tackifying resins include the pentaerythritol esters of tall oil, gum rosin, wood rosin or mixtures thereof.

Representative examples of such aliphatic resins include natural terpene resins, hydrogenated synthetic $C_9$ resins, hydrogenated synthetic $C_5$ resins, synthetic branched and unbranched $C_5$ resins, and mixtures thereof.

Representative examples of such aromatic-aliphatic tackifying resins include styrenated terpene resins, styrenated $C_5$ resins, or mixtures thereof.

The tendency of the adhesive to have rapidly changing properties or slowly changing properties relates to the compatibility of the tackifier/plasticizer combination. The variation in the compatibility of the components, primarily the tackifier (or tackifier blend) with the plasticizer, produces a variation in rate of change in properties to the equilibrium (ambient state) value. More compatible blends of tackifiers (or tackifier blends) and plasticizers tend to cause the properties to change (e.g., modulus increase) less rapidly. Less compatible blends result in more rapid change in properties. In other words, in a system containing largely aliphatic tackifying resins and aromatic tackifying resins with an aromatic plasticizer, increasing the proportion of aliphatic tacki-

11

fying resin will lead to an increase in the rate of modulus development. Some guidance regarding compatibility can be obtained from solubility parameter data. Compositions with more similar solubility parameters will tend to be more compatible, while large differences will tend to indicate less compatibility Additionally, the chemical nature of the materials can aid in selecting components for desired compatibility. Increasing aromaticity in the tackifier will tend to increase compatibility, while an increasing aliphatic nature will tend to reduce compatibility with the aromatic plasticizers of the invention.

Plasticizer

A plasticizer is broadly defined as a typically organic composition that can be added to rubbers and other resins to improve extrudability, flexibility, workability, or stretchability. Typical plasticizers in adhesives are plasticizing oils that are liquid at typical ambient temperature. The plasticizer used in the adhesives of the invention is typically a solid composition at ambient temperature having a softening point of at least 45° C.

Preferably, the plasticizer composition has a softening point of at least 60° C. Increased softening points (60-130° C.) can aid in improving heat resistance or preventing bond failure at high temperatures.

Two classes of plasticizers used in the invention generally comprise
(i) a diester of a cyclic dihydroxy substituted aromatic or aliphatic compound and a cyclic aliphatic or aromatic monocarboxylic acid compound; or
(ii) a diester of a cyclic dicarboxylic acid substituted aliphatic or aromatic compound and a cyclic hydroxy substituted aliphatic or aromatic compound.

One useful class of plasticizers used in the invention comprises a cyclo-aliphatic or aromatic ester of a benzene dicarboxylic acid. Such plasticiziers are prepared by forming an ester from a cyclo-aliphatic or aromatic alcohol such as cyclohexanol, phenol, naphthol, or other monohydroxy alcohol compounds having from 5 to 12 carbon atoms. The ester compounds are formed from dicarboxylic acid compounds, typically phthalic acids. Phthalic acids that can be used in the plasticiziers are 1,2-benzene dicarboxylic acid, 1,3-benzene dicarboxylic acid (isophthalic acid), or 1,4-benzene dicarboxylic acid (terephthalic acid). The preferred plasticizers of this class comprise dicyclohexyl phthalate or diphenyl phthalate. Most preferably, dicyclohexyl orthophthalate is used.

A second class of useful plasticizers comprise an aromatic carboxylic acid ester of a polyfunctional alcohol having 1 to 10 hydroxyl groups. Polyfunctional alcohols that can be used in the compositions of this class of plasticizers include compounds having at least two hydroxyl groups and at least two carbon atoms in the molecule. Specific examples of preferred hydroxy compounds include ethylene glycol, propylene glycol, 1,2-butylene glycol, 1,4-butylene glycol, glycerine, glucose, fructose, sucrose, mannitol, trimethylol ethane, 1,4-cyclohexane dimethanol, pentaerythritol, 2,2-dimethyl-1,3-propane diol, 2-hydroxy methyl-2-methyl-1,3-propane diol, neopentyl glycol, and other useful polyfunctional hydroxyl compounds. Aromatic acids that can be used with the polyfunctional alcohols to form this class ester plasticizer compounds of the invention include aromatic carboxylic acids, typically having at least one aromatic group and at least one carboxyl function. Representative acids include benzoic acid, naphthanoic acid, and 4-methyl benzoic acid. Typical examples of such useful plasticizers include triethylene glycol tribenzoate, trimethylol ethane tribenzoate, glycerol tribenzoate, sucrose benzoate, pentaerythritol tetrabenzoate, 2,2-dimethyl-1,3-propane diol dibenzoate, triethylene glycol dibenzoate, glycerol tribenzoate, 2-hydroxymethyl-2-methyl-1,3-propane diol tribenzoate pentaerythrithol tetrabenzoate, neopenyl glycol dibenzoate, mixtures thereof, and others.

The most preferred plasticizer is a solid with a softening point above 60° C. which belongs to the class of plasticizers including cyclohexane dimethanol dibenzoate compounds. A 1,4-cyclohexane dimethanol dibenzoate (containing cis- and trans- isomers) is exemplified and produces the maximum control over variation and change in adhesive physical properties.

A third class of useful plasticizers for use in the invention comprise a sulfonamide class made from aromatic sulfonic acids. Such plasticizers generally fall within the structural formula:

$$R\text{-}Ar\text{-}SO_2\text{-}NR_2$$

wherein each R is independently selected from the group consisting of hydrogen, aliphatic and cyclo-aliphatic radicals having 1 to 12 carbon atoms. Each R can be typically hydrogen, methyl, ethyl, propyl, isopropyl, n-butyl, isobutyl, tertiary butyl, ethyl hexyl, neopentyl, cyclohexyl, deodecyl, etc. R is preferably methyl, ethyl or cyclohexyl. Such sulfonamide plasticizers can also be used in the form of a resinous material formed through the condensation of formaldehyde with said sulfonamide plasticizer.

The Thermoplastic Polymer Base

The thermoplastic polymer base that can be used in manufacturing the novel adhesives of this invention are thermoplastic polymers that have sufficient compatibility with the tackifier, plasticizer, and oil components to form a homogenous melt and solid. In the adhesive of high polymer content, thermoplastic material provides to the adhesive sufficient cohesive strength such that the adhesive, after application and attainment of maximum modulus, forms a cohesively competent adhesive bonding mass.

In the low polymer content materials, the polymer acts to control viscosity and to produce initial bond values. The plasticized low polymer system tends to reduce the initial $T_g$ resulting in a composition that machines well and has good initial tack. After reaching equilibrium, the $T_g$ is no longer suppressed and the adhesive can solidify into a somewhat competent mass.

Any of a variety of available thermoplastic materials can be used in the compositions of the invention. Examples of such thermoplastics are ethylene based polymers such as ethylene/vinyl acetate, ethylene acrylate, ethylene methacrylate , ethylene methyl acrylate, ethylene methyl methacrylate, copolymers or ethylene and 1-6 mono- or di-unsaturated monomers, polyamides, polybutadiene rubber, polyesters such as polyethylene terephthalate, polybutylene terephthalate, etc., thermoplastic polycarbonates, atactic poly-alpha-olefins, including atactic polyethylene, atactic polypropylene, and others; thermoplastic polyacrylamides, polyacrylonitrile, copolymers or acrylonitrile with other monomers such as butadiene, styrene, etc., polymethyl pentene, polyphenylene sulfide, aromatic polyurethanes; styrene-acrylonitrile, acrylonitrile-butadiene-styrene, styrene-butadiene rubbers, polyethylene terephthalate, acrylonitrile-butadiene-styrene elastomers, polyphenylene sulfide. Also, A-B, A-B-A, A-(B-A)$_n$-B, (A-B)$_n$-Y block copolymers wherein the A comprises a polyvinyl aromatic block, the B block comprises a rubbery midblock, and others can be used. The aromatic character of the polymers provide compatibility with the aromatic plasticizing agents discussed below and provide controlled compatibility with the tackifier or the tackifier blends used to control modulus in the adhesive compositions. The preferred polymers should have a molecular weight sufficient that, when used in an adhesive formulation, the adhesive can maintain a high cohesive strength.

Additionally, we have found that ethylene vinyl acetate thermoplastic polymers, preferably having 5 to 50 wt-% vinyl acetate and a melt index of at least 10, are also preferred for use in this invention. While the ethylene/vinyl acetate polymers have no aromatic character, they form compatible hot melt adhesives having controlled modulus physical property changes when applied in the end uses disclosed.

In the case of ethylene/vinyl acetate thermoplastic copolymers, the variation in properties obtained through the plasticizer tackifier combinations of the invention causes the ethylene/vinyl acetate based materials to be fluid initially because of a substantially reduced $T_g$ which slowly increases over time until the $T_g$ reaches a point wherein the material becomes no longer a fluid. In these systems, the peel strength of the adhesive can drop substantially over time . Generally, after 10 minutes, 3 hours, or 6 hours, depending on formulation, the peel strength drops to less than 180 g/cm (one lb. per inch)

Other preferred polymers for use in the adhesives of this invention comprise linear A-B-A block, linear A-(B-A)$_n$-B multiblock copolymers, and radial or teleblock copolymers of the formula (A-B)$_n$-Y wherein A comprises a polystyrene block, B comprises a substantially rubbery polybutadiene or polyisoprene block, Y comprises a multivalent compound, and n is an integer of at least 3. The midblocks can be post-treated to improve their heat stability through hydrogenation or other post-treatment removing residual unsaturation. We believe that the size and the amount of the A or end blocks in the A-B-A block of copolymer structure should be as much as 15-51 wt-% of the polymer.

While the total styrene content of the polymers can be as much as 51 wt-% of the polymer, and since the polymers can have more than two A blocks or optional performance, the largest A block should be less than or equal to 20 wt-% of the polymer, and, most preferably, is less than or equal to 15 wt-% of the polymer. In an S-B-S (styrene-butadiene-styrene) copolymer, the preferred molecular weight is 50,000 to 120,000, and the preferred styrene content is about 20 to 35 wt-%. In an S-I-S (styrene-isoprene-styrene) copolymer, the preferred molecular weight is 100,000 to 150,000 and the preferred styrene content is 14-30 wt-%. Hydrogenating the butadiene midblocks produces rubbery midblocks that are typically considered to be ethylenebutylene midblocks.

Such block copolymers are available from Shell Chemical Company, Enichem and Fina. Multiblock or tapered block copolymers (the A-(B-A)$_n$-B type) are available from Firestone under the STEREON 840A and 845 trademarks.

In view of the disclaimer in Claim 1 for Contracting States  DE, GB, FR, IT, NL, SE, LI, CH, BE, AT and LU, the above-mentioned embodiments using linear A-B-A or A-(B-A)$_n$-B multiblock or tapered block copolymers do not generally form embodiments of the invention for these Contracting States.

The adhesive compositions of the invention can contain other compatible polymers, fillers, pigments, dyes, oils, catalysts, inhibitors, antioxidants, UV absorbers, waxes, and other conventional additives.

In construction methods using the adhesives of the invention, the adhesives are typically applied from applicators such as spray heads, print wheels, or extruders that can deliver the adhesive at elevated temperatures (typically above 121°C (250°F), and typically in the range of 135 - 205°C (275-400°F)). Extruder applicators can apply a bead of adhesive in any arbitrary width. The width selected typically obtains a high quality bond with minimal adhesive application. Print wheels apply the adhesive in a pattern to a surface at sufficient add-on quantities to obtain high bond strength at minimal adhesive application. The spray heads have apertures that range from 0.025 cm to 0.1 cm (0.01 to 0.04 inches). Under the operating conditions of typical adhesive spray machines, the diameter of the sprayed adhesive fiber can range from the size of the aperture to as little as about 0.0025 cm (0.001 inches) depending on operating conditions.

Depending on the end use and final bond strength desired, the adhesive can be applied at amounts that range from 0.08 mg/cm$^2$ (0.5 milligrams per square inch) to as much as 1.6 mg/cm$^2$ (10 milligrams per square inch). Extruded adhesive is applied at a rate of up to 6 mg/cm (15 mg/linear inch) or more. Preferably, because of the unique properties of the adhesives of this invention, the adhesives can be used at an application amount of from 0.08 mg/cm$^2$ (0.5 milligrams per square inch) to 1.6 mg/cm$^2$ (10 milligrams per square inch).

In the sealing of cigarette cartons , a low polymer adhesive composition of the invention is extruded in a bead or in discrete dots along a carton surface. The carton is closed by adhering a mating portion of the carton to the adhesive on the carton surface. Such low polymer adhesives maintain the integrity of the carton during manufacture but after storage, and attaining the equilibrium values of the adhesive composition, the carton is easily opened manually, because the adhesive becomes brittle and the bond obtains a low peel strength with substantial sheer strength.

Similarly, in the use of a palletizing adhesive, small amounts of adhesive are applied to units placed in a palletized assembly. The small amounts of adhesive maintain the mechanical pallet integrity during the construction of the pallet and, after a time, the adhesive obtains its equilibrium value, causing the peel strength of the adhesive to reduce substantially, rendering the pallet easily disassembled at the use site.

Similarly, removable coupons can be applied to containers using the low polymer adhesives of the invention. Such coupons are attached to the containers with great integrity during bottling and labeling. Once distributed and purchased at a distribution site, the labels are easily removed because the adhesives reached low equilibrium peel value and are easily removed from the container without damage to the coupon.

The adhesive compositions of the invention, particularly the high polymer compositions, can be used in the manufacture of disposable articles. The high polymer content adhesives have the ability to penetrate the nonwoven of the disposable article because of their low modulus after cooling. The adhesive, before reaching a high equilibrium modulus, appears to be a soft liquid material. The low modulus of the high polymer adhesives causes the material to display excellent wetting properties resulting in high quality bonds with great integrity. The low initial modulus of the adhesive of the invention permits operators to spray the high polymer adhesives at relatively low temperatures because the materials retain significant fluidity before the materials reach the final equilibrium modulus. This is in sharp contrast to conventional hot melt sprays which are typically used at very high temperatures in order to maintain sufficient fluidity for penetration of porous substrates.

During the manufacture of disposable articles using the adhesives of the invention, two modes of application are preferred. One mode of operation involves spraying the adhesive upon a fabric, such as a tissue, a woven or nonwoven web, or other material having permeability to the adhesive. Such sprayed-on adhesive can penetrate the permeable tissue, nonwoven or woven fiber, to cause the sheet to be embedded in the adhesive and adhered to the substrate such as an absorbent layer, back layer, or film. Alternatively, the adhesives of the invention can be directly applied to back sheet or film and the tissue, woven or nonwoven fabric, or other material can be applied to the adhesive on the film. The adhesive retains sufficient liquidity that it can penetrate pores or apertures in the fabric to form a mechanical bond. In the manufacture of tissue fluff absorbent cores, the fluff is typically wrapped by tissue. The tissue layer can be wrapped around the fluff and can overlap. Adhesive can then be sprayed on the overlapping portion of tissue outerwrap, can penetrate the wrappings and adhere the tissue to the fluff ensuring that the fluff obtains dimensional stability from adherence to the outer wrap.

In somewhat greater detail, the sprayable, hot melt adhesive compositions of the invention typically comprise an effective amount of a tackifying agent and sufficient solid plasticizers of desired degree of compatibility and an effective amount of a thermoplastic polymer base to form an effective adhesive that has the unique property that, after spraying and cooling, it can display a change in at least one physical property.

The hot melt adhesives of the invention are made in common hot melt manufacturing equipment. In the manufacture of the hot melt adhesives of the invention, the thermoplastic polymer is typically added to a melt comprising either the tackifier or the plasticizer material or mixtures thereof. Such additions facilitate the blending of the polymer into a smooth, uniform mixture. In such a manufacturing regimen, either the tackifier or the plasticizer or a portion thereof is added to the manufacturing equipment under inert atmosphere (with optional antioxidants) and is heated and agitated until melted. The thermoplastic polymer is then added to the melt at a rate such that the mixture forms a uniform smooth blend within a reasonable period. Antioxidant materials used in the manufacture of the adhesive can be added to the melt prior to, with, or after the addition of the polymer. Once a smooth blend of the polymer in conjunction with an adhesive component is formed, the balance of the components of the hot melt adhesives can be added at a convenient rate. Once the uniform blend of all the adhesive ingredients is formed, the adhesive can be drawn off and packaged in a convenient form including in drums , blocks, pillows, pellets, granules, etc. The following examples provide additional information with respect to the manufacture of the adhesives of the invention and include the best mode.

For Contracting States DE, GB, FR, IT, NL, SE, LI, CH, BE, AT and LU only Examples I -VII and XI do not necessarily form embodiments of the invention, in view of the disclaimer in Claim 1 for these Contracting States.

## Example I

Into a sigma blade mixer having a nitrogen atmosphere and heated to a temperature of 177°C (350°F) was added about 49.5 parts of styrenated terpene tackifying resin (ZONATAC 105) and 0.5 parts of an antioxidant (IRGANOX 1010). The mixer was operated until the antioxidant was fully blended with the molten tackifying resin. Into the tackifying resin was added 25 parts or a styrene-butadiene-styrene block copolymer having 37 wt-% styrene (KRATON D-1122, Shell Chemical Co.). After the copolymer had been fully added to the resin and a uniform melt resulted, 25 parts of a 1,4-dicyclohexane dimethanol dibenzoate (Benzoflex 352) were added. The sigma blade mixer was operated until the contents were a smooth melt blend and the material was withdrawn and packaged.

## Example II

Following the procedure of Example I, the following compositions were blended into a hot melt adhesive:

| Ingredient | Trade Name | Parts |
|---|---|---|
| S-B-S | KRATON 1122 | 25 |
| Antioxidant | IRGANOX 1330 | 0.5 |
| Tackifying agent | PERMALYN 603 | 49.5 |
| 1,4-Dicyclohexane-dimethanoldibenzoate | BENZOFLEX 352 | 25 |

## Example III

Following the procedure of Example I, the following compositions were blended into a hot melt adhesive:

| Ingredient | Trade Name | Parts |
|---|---|---|
| S-B-S | KRATON 1122 | 25 |
| Antioxidant | IRGANOX 1010 | 0.5 |
| Tackifying agent | PERMALYN 603/ | 24.75/ |
|  | ZONATAC 501 Blend | 24.75 |
| 1,4-Dicyclohexane-dimethanoldibenzoate | BENZOFLEX 352 | 25 |

Example IV

Following the procedure of Example I, the following compositions were blended into a hot melt adhesive:

| Ingredient | Trade Name | Parts |
|---|---|---|
| S-I-S | SOL T-193B | 30 |
| Antioxidant | IRGANOX 1010 | 0.5 |
| Tackifying agent | REGALREZ 1094 | 49.5 |
| 1,4-Dicyclohexane-dimethanoldibenzoate | BENZOFLEX 352 | 20 |

Example V

Example IV was repeated except that PERMALYN 603 was substituted for REGARLREZ 1094.

Example VI

Following the procedure of Example II, the following compositions were blended into a hot melt adhesive:

| Ingredient | Trade Name | Parts |
|---|---|---|
| S-E-B-S | KRATON G 1652 | 25 |
| Antioxidant | IRGANOX 1010 | 0.5 |
| Tackifying agent | REGALREZ 1094 | 49.5 |
| 1,4-Cyclohexane-dimethanoldibenzoate | BENZOFLEX 352 | 25 |

Example VII

Example VI was repeated except that PERMALYN 603 was substituted for REGARLEZ 1094.

Example VIII

Following the procedure of Example II, the following compositions were blended into a hot melt adhesive:

| Ingredient | Trade Name | Parts |
|---|---|---|
| E/VA (28% VA; MI = 20) | ELVAX 250 | 30 |
| Antioxidant | IRGANOX 1010 | 0.5 |
| Tackifying agent | PERMALYN 603 | 49.5 |

16

| 1,4-Cyclohexane- | | |
|---|---|---|
| dimethanoldibenzoate | BENZOFLEX 352 | 20 |

Example IX

Example VIII was repeated except that PERMALYN 603 was substituted for ZONATAC 501.

Example X

Following the procedure of Example XIII, the following compositions were blended into a hot melt adhesive:

| Ingredient | Trade Name | Parts |
|---|---|---|
| E/VA (28% VA; MI equals 20) | ELVAX 250 | 30 |
| Antioxidant | IRGANOX 1010 | 0.5 |
| Tackifying agent | PERMALYN 603/ | 49.5 |
| | ZONATAC 501 Light | |
| | Blend | |
| 1,4-Cyclohexane- | | |
| dimethanoldibenzoate | BENZOFLEX 352 | 20 |

Example XI

Following the procedure of Example I, the following compositions were blended into a hot melt adhesive:

| Ingredient | Trade Name | Grams |
|---|---|---|
| S-B-S/S-I-S Blend | KRATON 1102 & | 5/14 |
| | KRATON 1117 | |
| Antioxidant | ETHANOX 330 | 1.0 |
| Tackifying agent | ZONATAC 501 | 59 |
| 1,4-Dicyclohexane- | | |
| dimethanoldibenzoate | BENZOFLEX 352 | 21 |

Example XII

Fugitive Palletizing Hot Melt Adhesive Generally Following the Procedure of Example I

The following compositions were blended into a hot melt adhesive:

| Ingredient | Trade Name | Parts |
|---|---|---|
| Coumarin-Indene Tackifying Resin | NEVEX 110 | 40.9 |
| Black Colorant | VINSOL Resin | 2.0 |
| Antioxidant | IRGANOX 1076 | 1.0 |
| Ethylene Vinyl Acetate Resin | ELVAX 150 | 10.0 |
| Plasticizing Agent | BENZOFLEX 352 | 45.0 |

* ELVAX 150, 33 wt-% vinyl acetate and 40 melt index

Example XIII

Fugitive Palletizing Hot Melt Adhesive Generally Following the Procedure of Example I

The following compositions were blended into a hot melt adhesive:

| Ingredient | Trade Name | Parts |
|---|---|---|
| Tackifying Resin | ESCOREZ 7312 | 48.0 |
| Antioxidant | IRGANOX 1076 | 1.0 |
| Ethylene Vinyl Acetate Resin | ELVAX 150 | 10.0 |
| Plasticizing Agent | BENZOFLEX 352 | 39.9 |

The product. of Example XIII was prepared and extruded onto a corrugated paperboard substrate in a uniform line of about 0.5 cm (3/16") diameter. Additional pieces of the corrugated paperboard substrate were then placed over the glue line after an open time of 5 minutes. The additional substrate layers were compressed with a roller. The pieces were then peeled from the adhesive line at time intervals of 1 hour. Upon the application of substantial peel force, the substrates were removed only with substantial force and straining of the adhesive. After 4 hours, the substrate was removed with little peel force with no fiber tearing or straining of the adhesive.

A second set of experiments were done with the adhesive of Example XIII. A 0.5 cm (3/16") diameter bead was applied to a corrugated substrate. A second corrugated sheet was contacted with the bead. The corrugated sheet was scored with a blade, creating a 90 degree flap of board. The assembled board substrates were inserted with a peel tester and pulled apart at 98.4 m/min (300 ft/min). A duplicate set of assembled substrates were made. The first set were tested within 1 hour of preparation and showed strong fiber treating bonds of about 1.1 to 1.8 kg/cm (6 to 10 lb/inch). The second set were pulled at 15 hours after preparation and failed adhesively at about 0.18 to 0.27 kg/cm (1 to 1.5 lb/inch)

The modulus of the comparative Example A shown in the bottom of Table I is at its maximum value at the earliest time wherein the modulus can be measured after the adhesive is tested. In sharp comparison to the modulus of comparative Example A, the modulus of Examples 1-10 shows more or less rapid increase in modulus over the testing period. Note the format of the data, for example 2.4 E + 0.8 is representative of number

$2.4 \times 10^8$.

Example XIV (without thermoplastic polymer)

Into a 400 mL beaker was placed 90 grams of a 1.4 cyclohexanol dimethanol debenzoate (Benzoflex 352) plasticizer and 210 grams of a rosin ester (Permalyn 603) tackifier. The beaker and its contents were heated to a temperature of 177°C (350 degrees F) until melted. The melt was mixed until uniform.

A thin film of the adhesive was drawn onto a paper substrate and a second paper was bonded to the adhesive film. Immediately after application of the second substrate the adhesive bond exhibited substantial peel strength resulting in paper tearing bonds. Within twenty minutes of application, the adhesive exhibited little peel strength and failed with the application of moderate force in the peel mode. The substrate was unaffected in the application of the peel force. The adhesive had some shear strength remaining after twenty minutes.

Such an adhesive is suited for palletizing adhesive or label pickup adhesive applications because the adhesive has significant peel strength when applied but has little peel strength after a period of time. In palletizing adhesives, the adhesive needs to bond the pallet during formation but when the pallet is delivered and disassembled the adhesive should have minimal peel strength to promote the removal of the units of the pallet. Such an adhesive is also useful in label pickup adhesive applications.

These data clearly indicate that the selection of thermoplastic block copolymer (if any), tackifier, for tackifier blend, and plasticizer can exert significant and important control over a storage modulus of the adhesive.

Without wishing to restrict the invention to any theoretical explanation, it appears on the basis of present knowledge that the specific and unique properties of the adhesives according to this invention are related to the crystallization behaviour of the adhesive after application. More specifically, it is assumed that the interaction between the tackifier and the plasticizer components influence the crystallization rate, especially of the plasticizer component. It is further assumed that the variation of storage modulus, peel strength and shear strength after application are strongly influenced by the more or less delayed onset of crystallization, the final ambient state crystallization attained also influencing the final ambient state properties of the adhesive.

The tackifier/plasticizer combinations listed in table III were prepared to determine the effect of compatibility of the materials on change in modulus after cooling. These examples and the change in their properties in a heating or cooling cycle were investigated with differential scan calorimetry (DSC).

## TABLE III
### DSC
### Examples
### (wt-%)

|  | PERMALYN 603 | WT 95 | BENZOFLEX 352 | Oil |
|---|---|---|---|---|
| DSC 1 | 57 | 0 | 29 | 14 |
| DSC 2 | 43 | 14 | 29 | 14 |
| DSC 3 | 28.5 | 28.5 | 29 | 14 |
| DSC 4 | 14 | 43 | 29 | 14 |
| DSC 5 | 0 | 57 | 29 | 14 |
| DSC 6 | 0 | 0 | 100 | 0 |

DSC is a technique that follows the thermal changes associated with thermal transitions, such as curing, melting, crystallization, etc. Initially, four scans were made on each sample. The first was a dynamic heating scan at 10° per minute over the temperature range, -42 i 150°C. The second run was a programmed cooling scan made at 5° per minute from 150 to -40°C. The third run was a reheating scan of the material in the second run made again at 10° per minute over the range -40 to 150°C. The sample was then rapidly cooled from 150°C. at 200° per minute, and a fourth heating run of the quenched, cooled sample was made at 10° per minute over the range of -40 to 150°C.

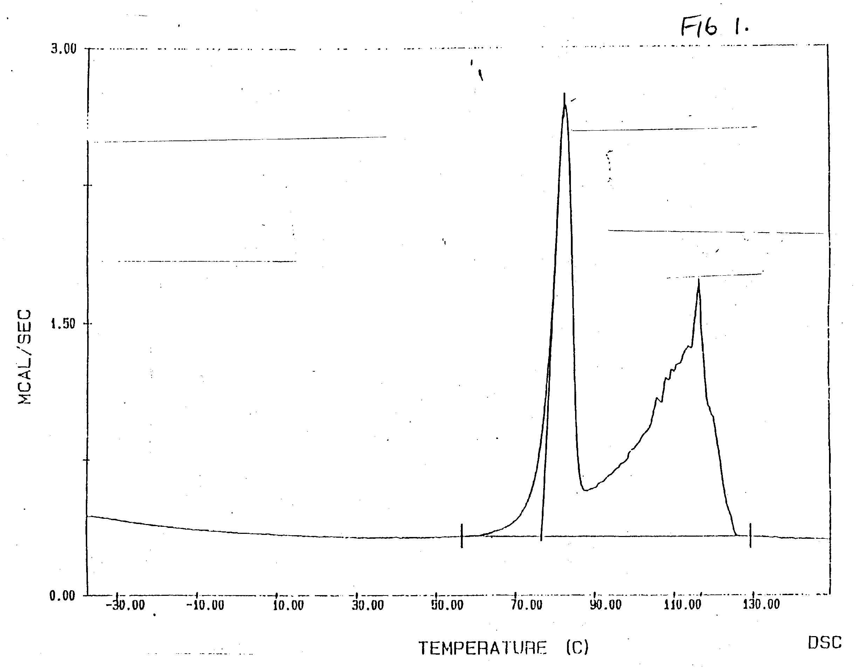

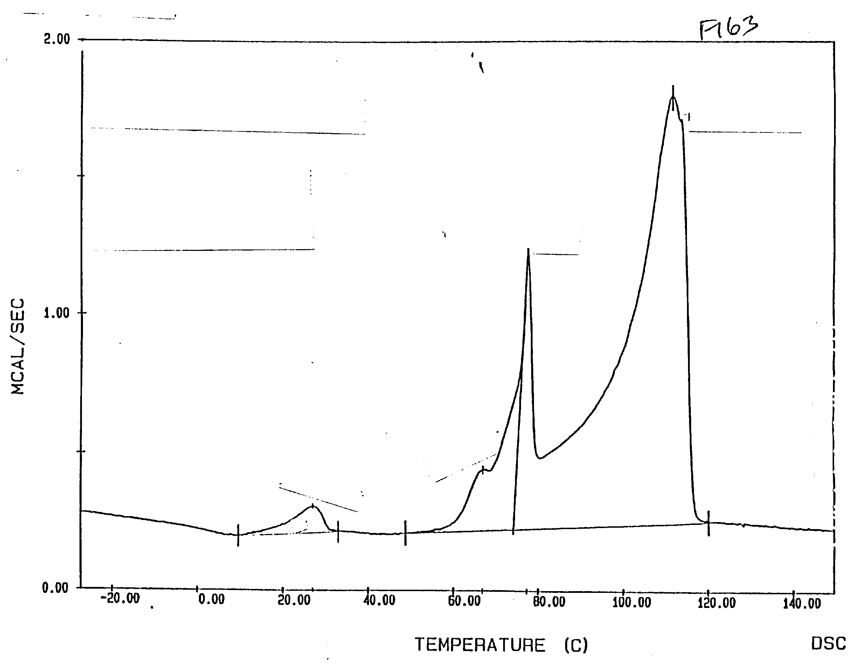

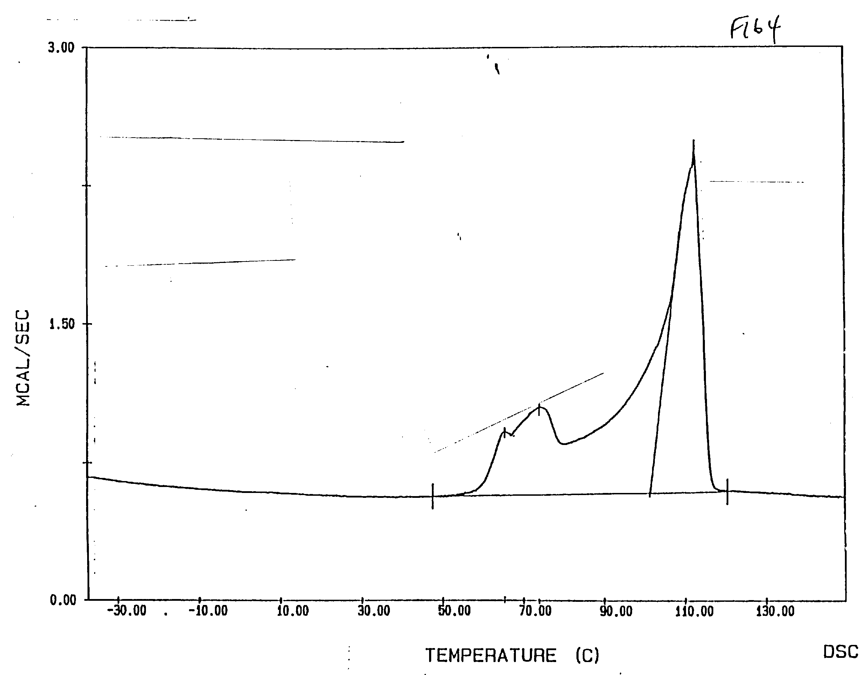

The first run is a melt profile of the sample which reflects the change in modulus caused by crystal growth associated with the previous cooling history. The second run is an attempt to follow the crystallization of the sample as it is slowly cooled. The third run as made to determine the effect the slow cooling had on the sample properties. The fourth run was made after the sample was quickly cooled to the amorphous state in order to limit crystallization. Figures 1 through 4 are the four runs discussed above for DSC 6 100% Benzoflex 352. These scans indicate that Benzoflex 352 readily crystallizes under normal conditions. The remaining figures are scans of blended materials showing the effect of the presence of the tackifier and oil in the compositions on crystal formation.

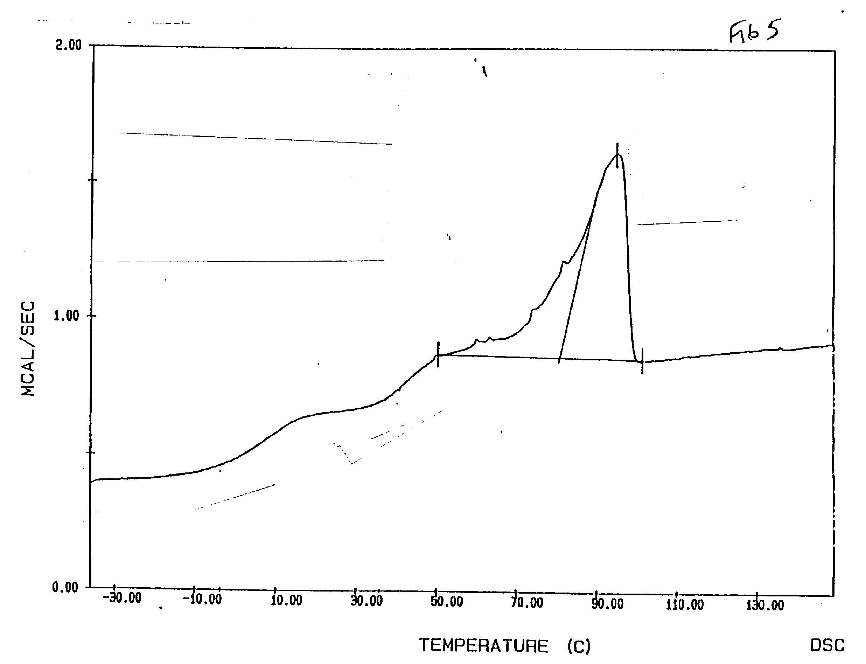

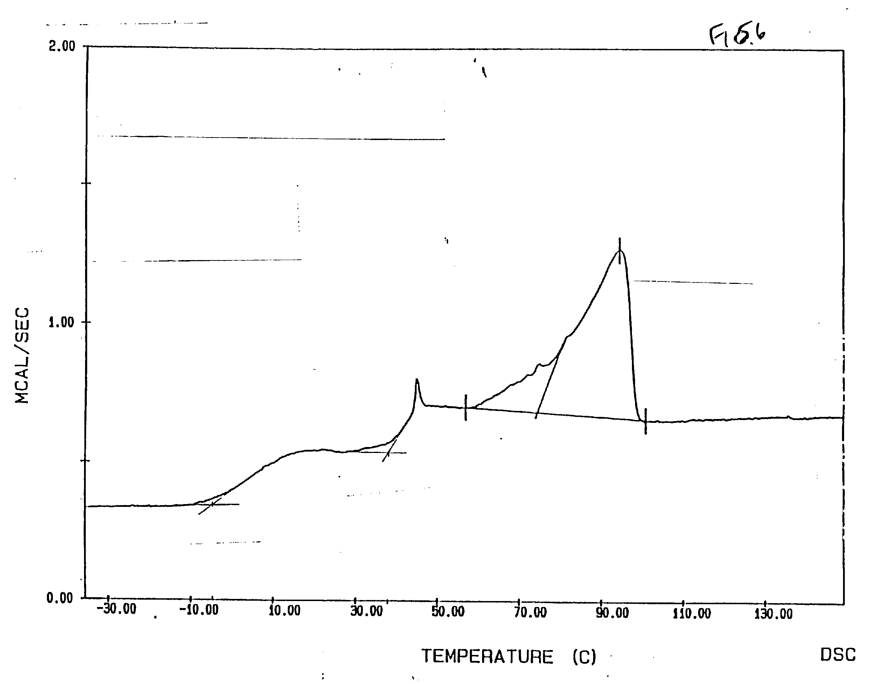

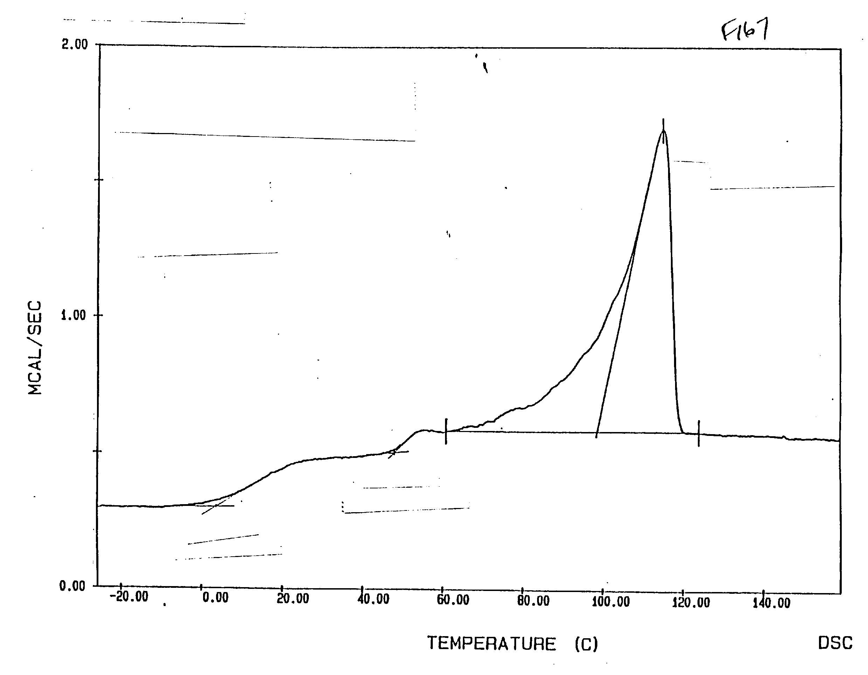

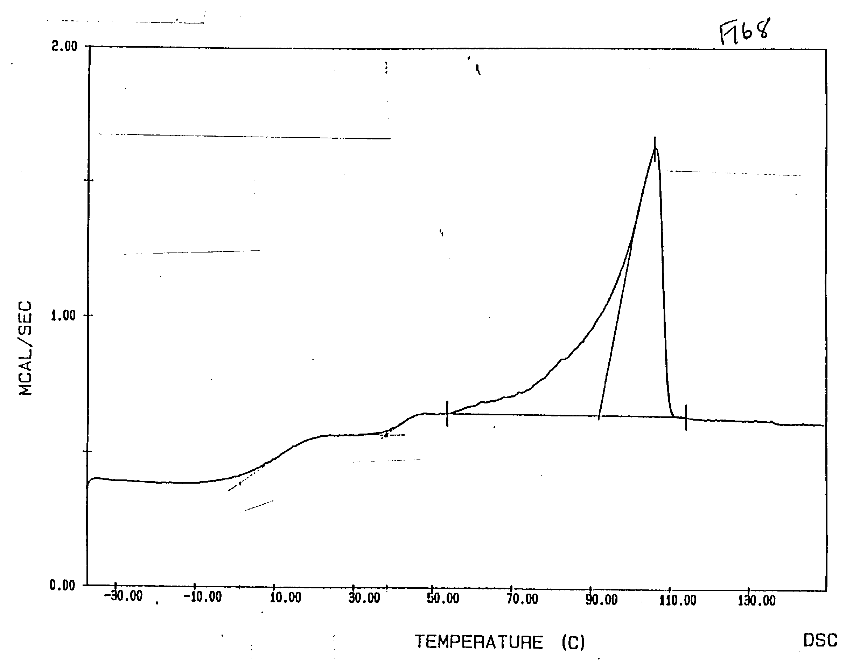

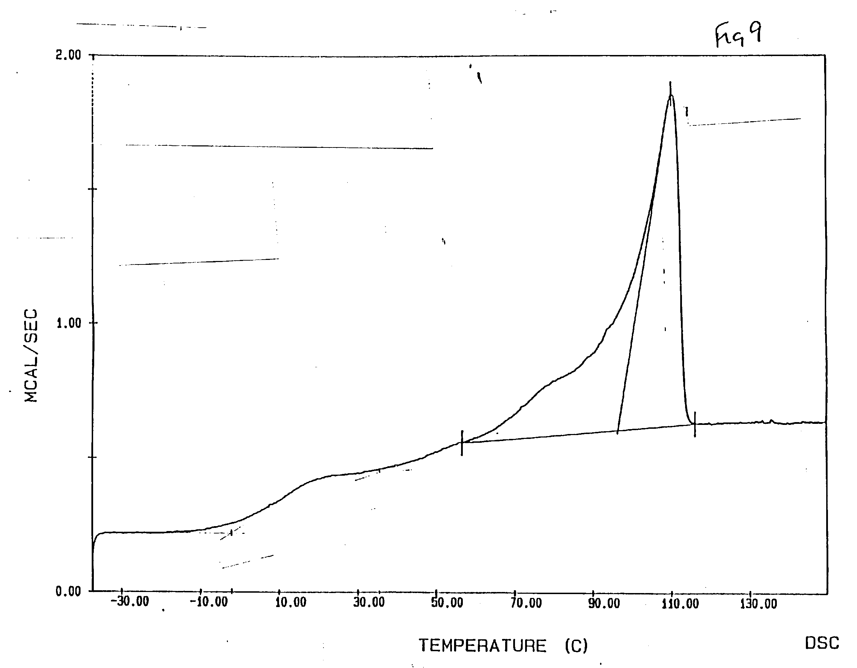

Figures 5 through 9 are the first run profiles for DSC 1 through 5. Each scan exhibits two glass transitions ($T_g$'s) and a single melt endotherm. These curves for the formulated materials are distinguished from the Benzoflex curve by the presence of two glass transition temperatures, the total amount of heat fusion required for the melt, and the temperature of the peaks on the scan. Clearly, when heated in a dynamic mode, the material melts in a substantially different mode from the pure plasticizer material.

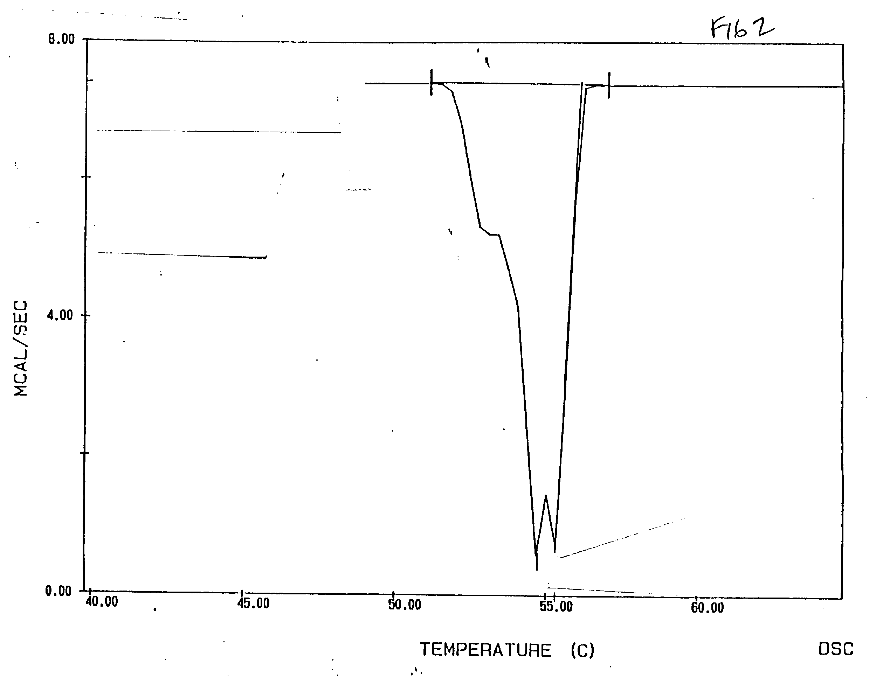

The second DSC runs, for which there are no figures showing the scans of a programmed cooling run, notes no crystallization of any hot melt samples. This is in sharp contrast with the crystallization noted in Figure 2 of Benzoflex 352.

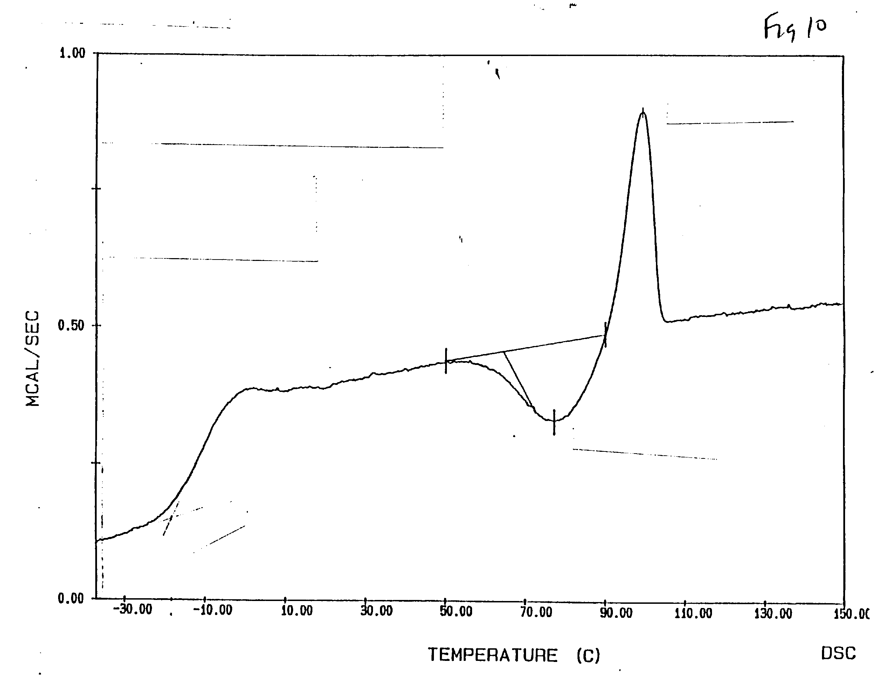

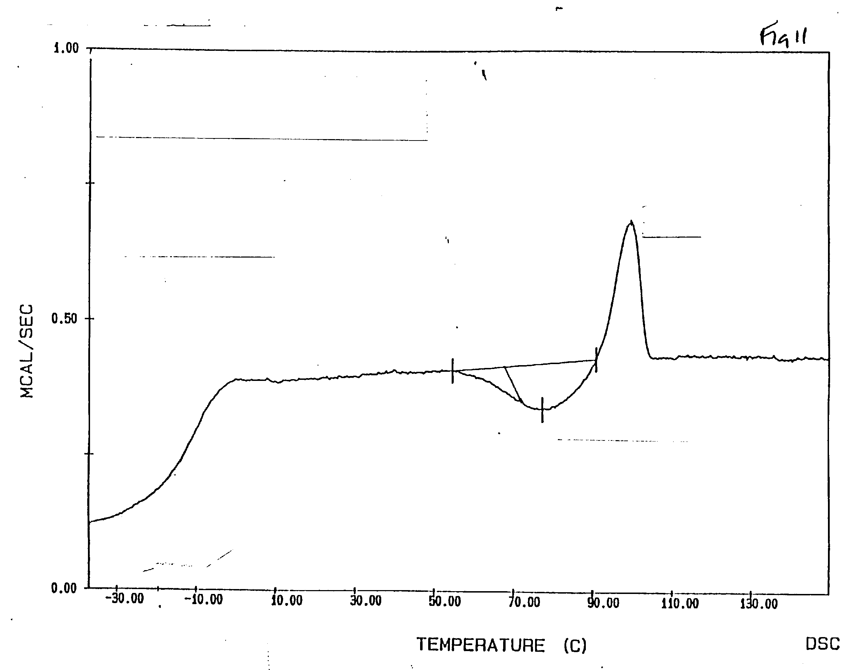

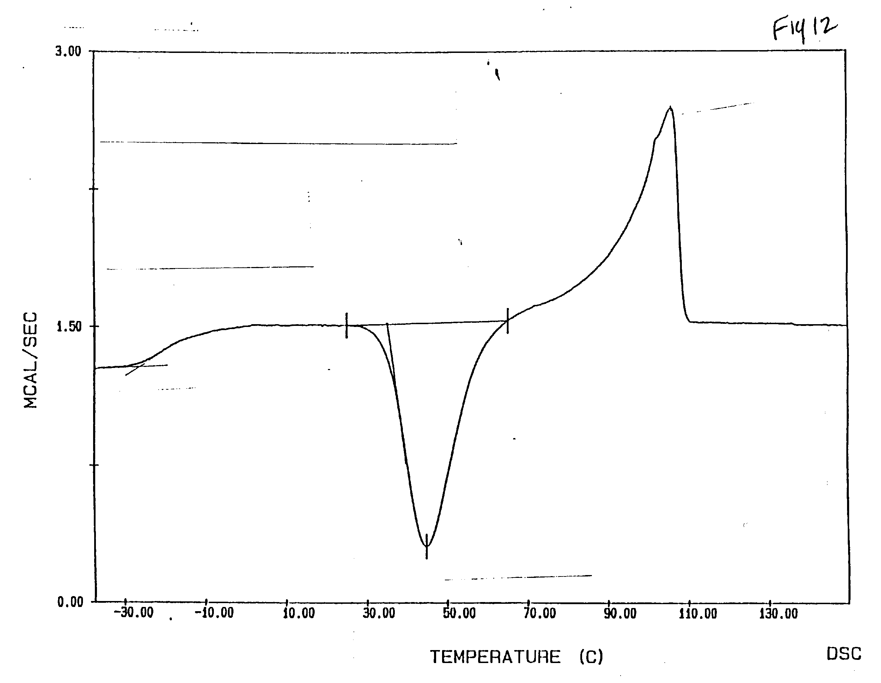

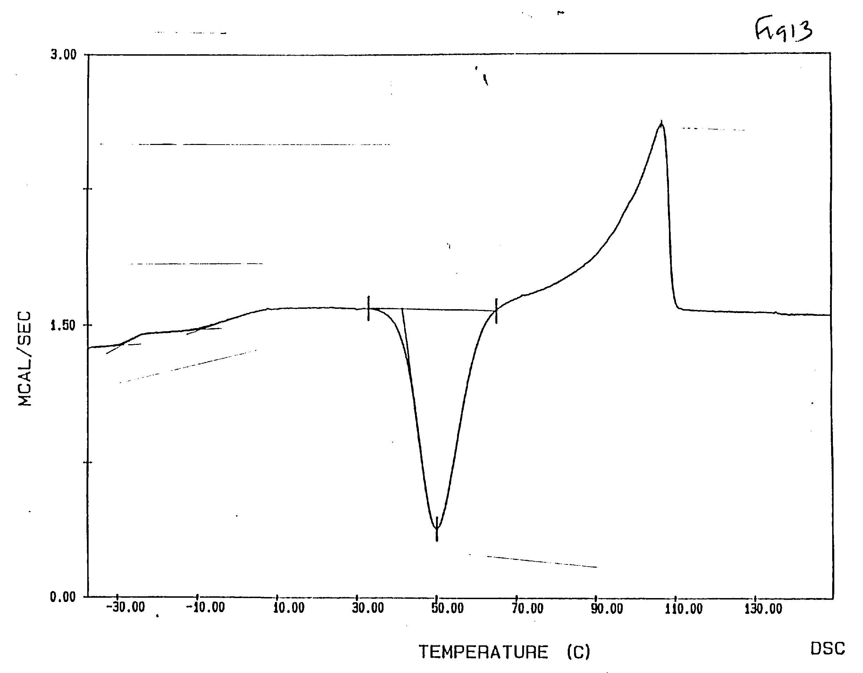

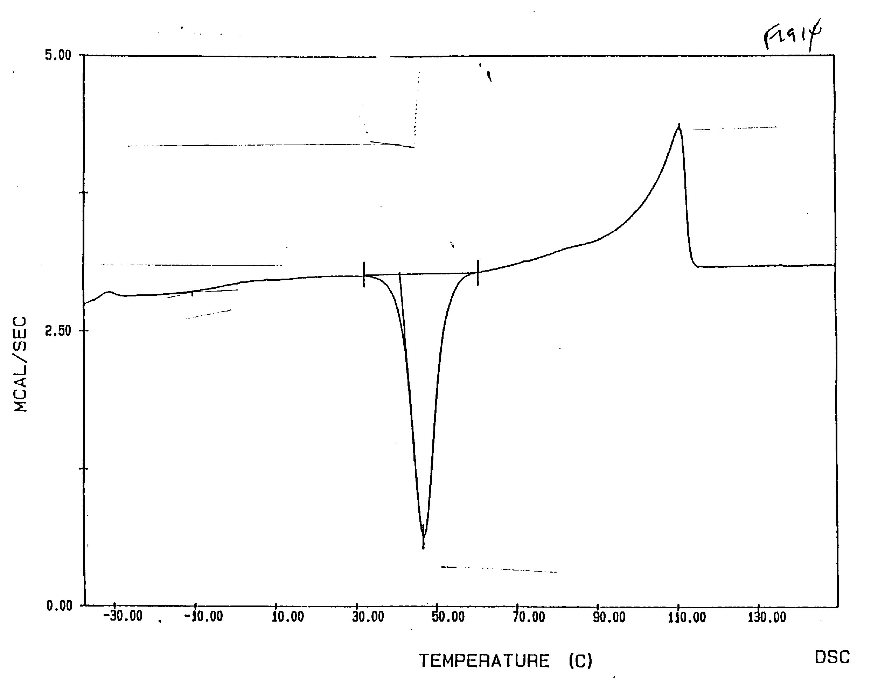

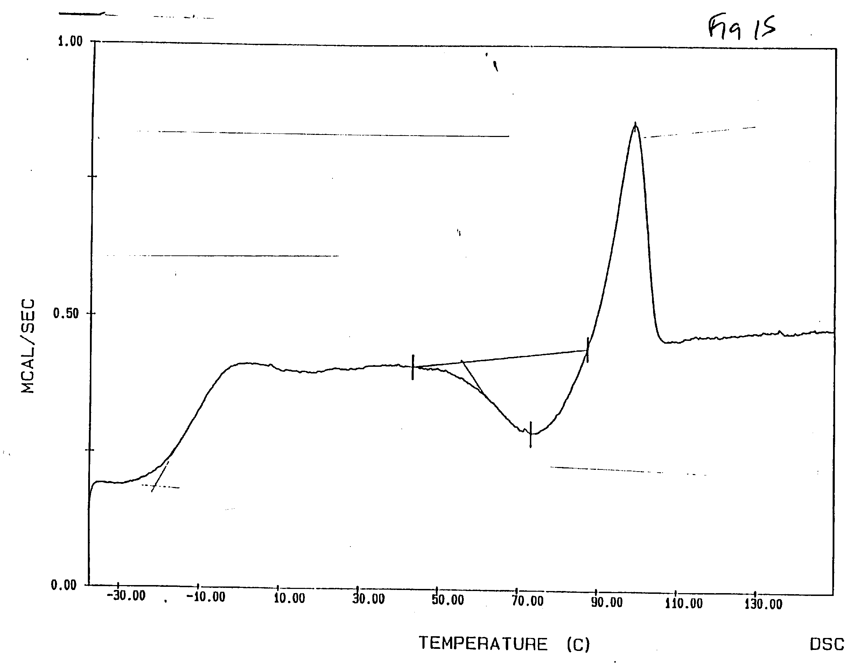

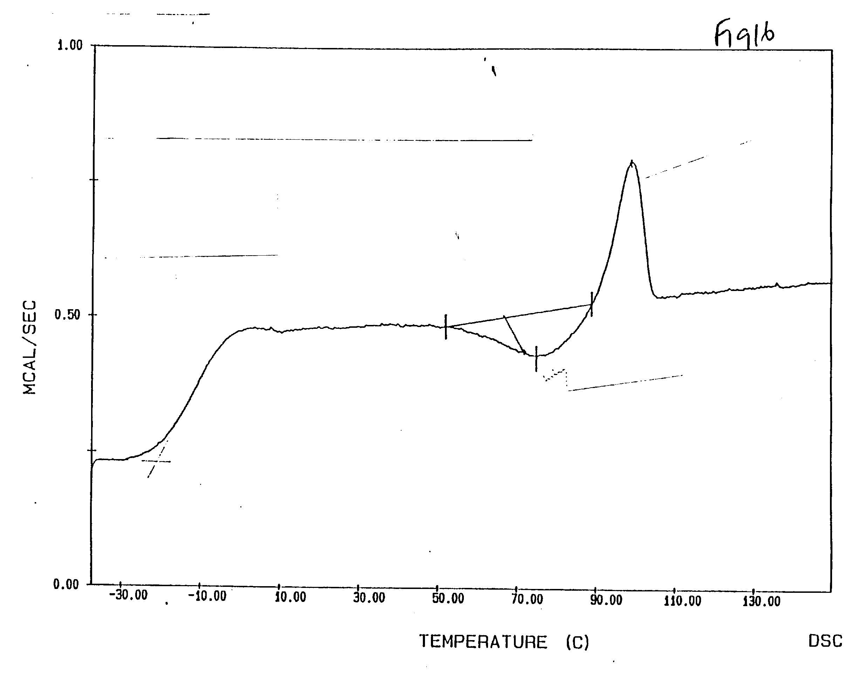

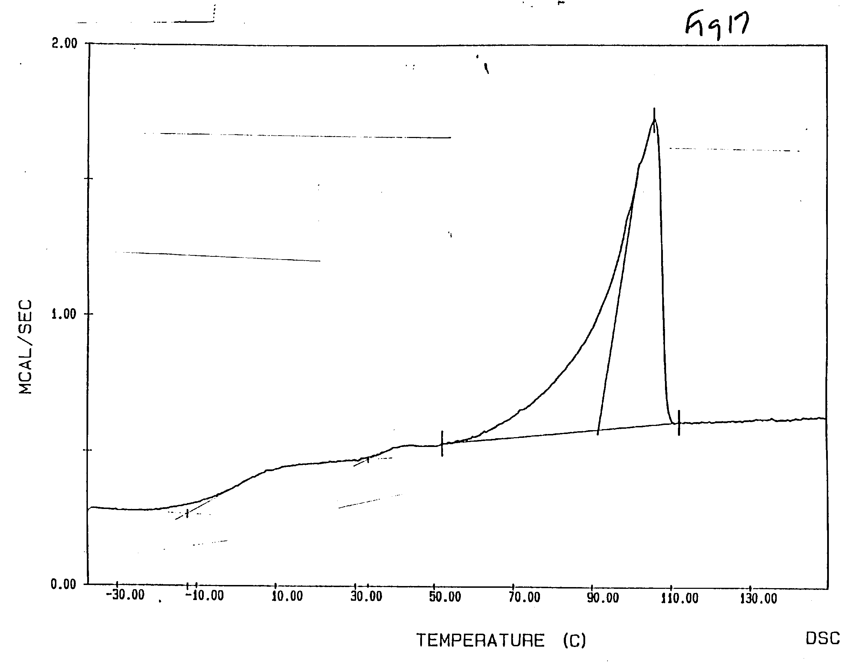

Figures 10 through 14 show an exothermic crystallization peak that immediately precedes an endothermic melting peak in the DSC run as discussed above. These scans show a cold crystallization phenomenon. Cold crystallization is the phenomenon observed when a molecule, generally polymeric molecule, is cooled quickly enough past its crystallization temperature and is frozen or super cooled in an amorphous state. Thermodynamically, these systems prefer particular order and, upon heating, reach a temperature where there is sufficient energy and mobility to rearrange into a crystal structure. The exotherms show such a cold crystallization. This cold crystallization shows that the resin oil combinations hinder crystallization considerably and effects a change in modulus for a period of time after the sample reaches ambient temperature. In order to determine the time period required for the hot melt samples to reach full modulus with full crystallization at 25°C., the samples were quenched cooled from 150° to 25°C. at 200° per minute and allowed to crystallize at 25°C. for various times, namely, 1 hour, 1.5 hours, 2 hours, and 5 hours. A melt profile was then run for each sample, after its respective times, and the resulting curves were evaluated. Figures 15 and 16 show that the samples DSC 1 and DSC 2 did not fully crystallize after even 5 hours as evidenced by the cold crystallization exotherms in the scan. Sample DSC 3 showed complete crystallization after 1 hour. Figure 17 displays no cold crystallization exotherm before the endotherm peak.

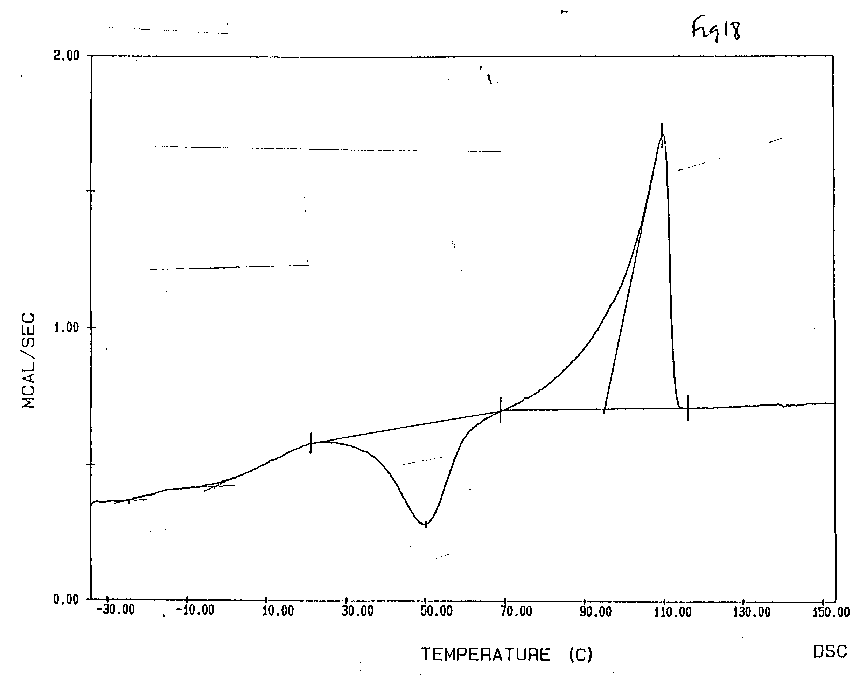

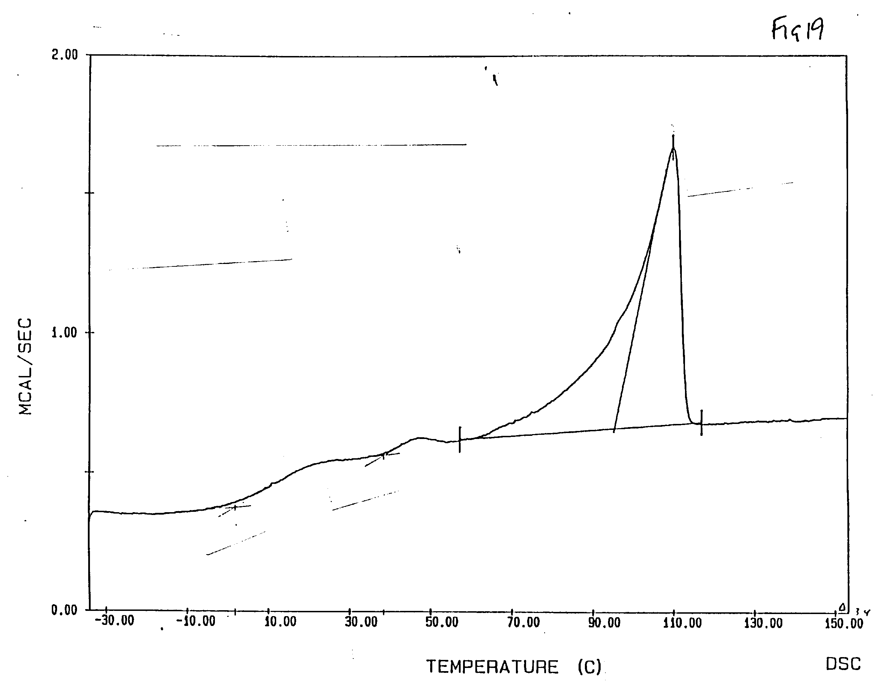

Figures 18 and 19 show that sample DSC 4 crystallizes between 1 hour and 1.5 hours. The 1 hour curve on Figure 18 has an exotherm showing that the sample is not fully crystallized, while Figure 19 shows full crystallization.

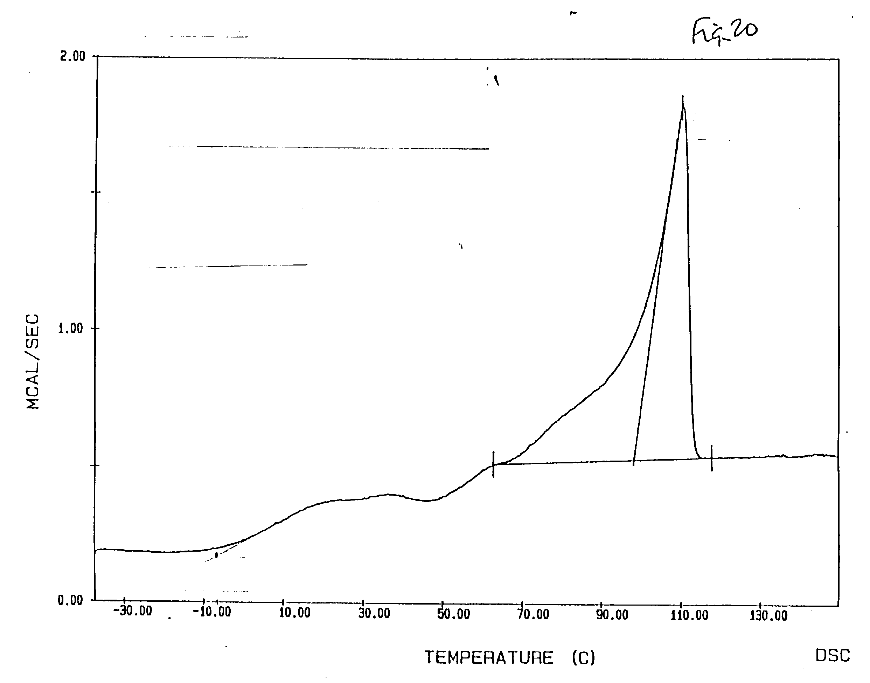

Figure 20 shows that sample DSC 5 is fully crystallized after 1 hour. From these curves, it is clear that the time for crystallization and the associated increase in modulus can be controlled through careful blending of components and control over ingredient proportions.

## Claims

### Claims for the following Contracting States : AT, BE, CH, DE, FR, GB, IT, LI, LU, NL, SE

1. A hot melt adhesive composition comprising:
   (a) 5 to 50 weight-% of a plasticizer, said plasticizer being a solid at room temperature and having a softening point of at least 45°C;
   (b) 20 to 80 weight-% of a tackifier selected from the group consisting of an aromatic, aromatic-aliphatic, aliphatic or a rosin tackifying resin; and
   (c) 0 to 55 weight-% of a thermoplastic polymer excluding thermoplastic block copolymers consisting of an A-B-A block copolymer, an A-(B-A)$_n$-B-A block copolymer, wherein n is an integer of 1 or more, or a radial block copolymer, each A comprising a polystyrene block and each B comprising a rubbery block.
   wherein the composition, after application and cooling, does not attain an ambient state for a physical property selected from the group consisting of storage modulus (G'), peel strength and shear strength for at least five minutes.

2. The composition of Claim 1, said plasticizer being a diester which comprises a diester of a cyclic dihydroxy substituted aromatic or aliphatic compound and a cyclic aliphatic or aromatic mono-carboxylic acid compound; or a diester of a cyclic dicarboxylic acid substituted aliphatic or aromatic compound and a cyclic hydroxy substituted aliphatic or aromatic compound.

3. The composition of Claim 2, wherein the plasticizer comprises an aromatic carboxylic acid ester of a polyfunctional alcohol having 1 to 10 hydroxyl groups, preferably of a dihydroxy cyclohexane compound and especially of a 1,4-substituted cyclohexane compound.

4. The composition of Claim 3, wherein the plasticizer is a cyclohexane dimethanol dibenzoate compound, especially 1,4-cyclohexane dimethanol dibenzoate.

5. The composition of any one of Claims 1 to 4, wherein the plasticizer comprises a mixture of cis- and trans-isomers.

6. The composition of any one of Claims 1 to 5, wherein the plasticizer has a softening point of at least 60°C.

7. The composition of Claim 1, wherein the plasticizer comprises a cyclo-aliphatic or aromatic ester of a benzene dicarboxylic acid, preferably a benzene-1,4-dicarboxylic acid and especially dicyclohexylorthophthalate or diphenylorthophthalate.

8. The composition of Claim 1, wherein the plasticizer comprises an aromatic sulfonamide compound, corresponding to the general formula:

$$R - Ar - SO_2 - NR_2,$$

wherein each R is independently selected from the group consisting of H, aliphatic and cycloaliphatic radicals having 1 to 12 carbon atoms, R being preferably methyl, ethyl or cyclohexyl; and/or comprises the formaldehyde condensation product of such sulfonamide plasticizer compounds.

9. The composition of any one of Claims 1 to 9, wherein the thermoplastic polymer is selected from ethylene based polymers such as ethylene/vinyl acetate, ethylene acrylate, ethylene methacrylate, ethylene methylacrylate, ethylene methyl methacrylate, copolymers of ethylene and 1-6-mono or di-unsaturated monomers, polyamides, polybutadiene rubber, polyesters such as polyethylene therephthalate, polybutylene therephthalate, thermoplastic polycarbonates, atactic poly-alpha-olefins including atactic polyethylene, atactic polypropylene and others; thermoplastic polyacrylamides, polyacrylonitrile, copolymers of acrylonitrile with other monomers such as butadiene, styrene etc., polymethyl pentene, polyphenylene sulfide, aromatic polyurethanes; styreneacrylonitrile, acrylonitrile-butadiene-styrene, styrenebutadiene rubbers, polyethylene therephthalate, acrylonitrile-butadiene-styrene elastomers and polyphenylene sulfide.

10. The composition of any one of Claims 1 to 9, wherein the thermoplastic polymers is present at a concentration of 5 to 40 weight-%.

11. The composition of Claim 9 or 10, wherein at least after 5 minutes after the composition is applied and cooled, the adhesive exhibits at least 10 fold increase in storage modulus over the modulus of the composition when first cooled.

12. The composition of Claim 9, 10 or 11 comprising:
(a) 5 to 50 weight-% of a cyclohexane dimethanol dibenzoate plasticizer;
(b) 20 to 80 weight-% of a tackifier selected from the grouop consisting of an aromatic, aromatic-aliphatic, aliphatic or rosin tackifying resin; and
(c) 10 to 55 weight-% of a thermoplastic block copolymer;
wherein the composition, after application, does not attain a final equilibrium value for a physical property selected from the group of properties consisting of storage modulus (G'), peel strength or shear strength for 5 to 10 minutes after application at ambient temperatures at the workplace.

13. The composition of any one of Claims 1 to 9 comprising
(a) 5 to 50 weight-% of a plasticizer which comprises a diester of a cyclic dihydroxy substituted aromatic or aliphatic compound and a cyclic aliphatic or aromatic mono-carboxylic acid compound; or a diester of a cyclic dicarboxylic acid substituted aliphatic or aromatic compound and a cyclic hydroxy substituted aliphatic or aromatic compound, said plasticizer being a solid at room temperature and having a softening point of at least 45°C;

(b) 20 to 80 weight-% of a tackifier selected from the group consisting of an aromatic, aromatic-aliphatic, aliphatic or a rosin tackifying resin; and

(c) optionally up to 15 weight-% of a thermoplastic polymer;

said hot melt composition being formulated to form, upon application, a fugitive cohesive bond which is initially strong under shear and peel, said bond changing with time to a brittle bond that has significant shear strength but little peel strength.

14. The composition of Claim 13, wherein the thermoplastic copolymer comprises an ethylene-vinyl acetate polymer.

15. The composition of Claim 14, wherein the composition has an initial peel strength immediately after application of at least 1.1 kg/cm (6 lb./inch) and final equilibrium peel strength of less than 0.18 kg/cm (1 lb./inch).

16. The composition of any one of Claims 13 to 15 comprising:

(a) 5 to 40 weight-% of a cyclohexane dimethanol dibenzoate plasticizer compound;

(b) 20 to 80 weight-% of a tackifier selected from the group consisting of an aromatic, aromatic-aliphatic, aliphatic or rosin tackifying resin and

(c) 0 to 15 weight-% of a thermoplastic ethylene vinyl acetate copolymer;

wherein the composition, after application, does not attain a final equilibrium bond peel strength for at least 30 minutes after application.

17. A case or carton, especially a cigarette carton secured by an effective amount of the hot melt adhesive according to any one of Claims 13 through 16.

18. A pallet comprising an assembly of cases or cartons secured by an effective amount of the hot melt adhesive according to any one of Claims 13 to 16.

19. A coupon or a label removably adhesively attached by means of a hot melt adhesive composition according to any one of Claims 13 through 16.

20. A disposable article comprising an absorbent layer adhered to a substrate, especially an outer wrap, by the adhesive according to any one of Claims 1 through 12.

21. The article of Claim 20, wherein the article is a feminine pad.

22. The article of Claim 20, wherein the article is a disposable diaper.

23. The article of any one of Claims 20 through 22, the composition being sprayed on the layer and/or the substrate.

**Claims for the following Contracting States : DK, ES, GR**

1. A hot melt adhesive composition comprising:

(a) 5 to 50 weight-% of a plasticizer said plasticizer being a solid at room temperature and having a softening point of at least 45°C;

(b) 20 to 80 weight-% of a tackifier selected from the group consisting of an aromatic, aromatic-aliphatic, aliphatic or a rosin tackifying resin; and

(c) 0 to 55 weight-% of a thermoplastic polymer;

wherein the composition, after application in cooling, does not attain an ambient state for a physical property selected from the group consisting of storage modulus (G'), peel strength and shear strength for at least five minutes.

2. The composition of Claim 1, said plasticizer being a diester which comprises a diester of a cyclic dihydroxy substituted aromatic or aliphatic compound and a cyclic aliphatic or aromatic mono-carboxylic acid compound; or a diester of a cyclic dicarboxylic acid substituted aliphatic or aromatic compound and a cyclic hydroxy substituted aliphatic or aromatic compound,

3. The composition of Claim 2, wherein the plasticizer comprises an aromatic carboxylic acid ester of a polyfunctional alcohol having 1 to 10 hydroxyl groups, preferably of a dihydroxy cyclohexane compound and especially of a 1,4-substituted cyclohexane compound.

4. The composition of Claim 3, wherein the plasticizer is a cyclohexane dimethanol dibenzoate compound, especially 1,4-cyclohexane dimethanol dibenzoate.

5. The composition of any one of Claims 1 to 4, wherein the plasticizer comprises a mixture of cis- and trans-isomers.

6. The composition of any one of Claims 1 to 5, wherein the plasticizer has a softening point of at least 60°C.

7. The composition of Claim 1, wherein the plasticizer comprises a cyclo-aliphatic or aromatic ester of a benzene dicarboxylic acid, preferably a benzene-1,4-dicarboxylic acid and especially dicyclohexylorthophthalate or diphenylorthophthalate.

8. The composition of Claim 1, wherein the plasticizer comprises an aromatic sulfonamide compound, corresponding to the general formula:

$$R - Ar - SO_2 - NR_2,$$

wherein each R is independently selected from the group consisting of H, aliphatic and cycloaliphatic radicals having 1 to 12 carbon atoms, R being preferably methyl, ethyl or cyclohexyl; and/or comprises the formaldehyde condensation product of such sulfonamide plasticizer compounds.

9. The composition of any one of Claims 1 to 8, wherein the thermoplastic polymer is present at a concentration of 5 to 40 weight-%.

10. The composition of Claim 9, wherein the thermoplastic polymer comprises a block copolymer, preferably an A-B-A block copolymer, wherein A comprises a styrene block and B comprises a rubbery block.

11. The composition of Claim 9 or 10, wherein at least after 5 minutes after the composition is applied and cooled, the adhesive exhibits at least 10 fold increase in storage modulus over the modulus of the composition when first cooled.

12. The composition of Claim 10 or 11 comprising:
    (a) 5 to 50 weight-% of a cyclohexane dimethanol dibenzoate plasticizer;
    (b) 20 to 80 weight-% of a tackifier selected from the grouop consisting of an aromatic, aromatic-aliphatic, aliphatic or rosin tackifying resin; and
    (c) 10 to 55 weight-% of a thermoplastic block copolymer, especially an A-B-A block copolymer wherein each A comprises a styrene block and each B comprises a rubbery block copolymer;
    wherein the composition, after application, does not attain a final equilibrium value for a physical property selected from the group of properties consisting of storage modulus (G'), peel strength or shear strength for 5 to 10 minutes after application at ambient temperatures at the workplace.

13. The composition of any one of Claims 1 to 9 comprising
    (a) 5 to 50 weight-% of a plasticizer, said plasticizer being a solid at room temperature and having a softening point of at least 45°C;
    (b) 20 to 80 weight-% of a tackifier selected from the group consisting of an aromatic, aromatic-aliphatic, aliphatic or a rosin tackifying resin; and
    (c) optionally up to 15 weight-% of a thermoplastic polymer;
    said hot melt composition being formulated to form, upon application, a fugitive cohesive bond which is initially strong under shear and peel, said bond changing with time to a brittle bond that has significant shear strength but little peel strength.

14. The composition of Claim 13, wherein the thermoplastic polymer is present at a concentration of 0.1 to 10 weight-%.

15. The composition of any one of Claims 1 to 8, wherein the thermoplastic polymer is selected from ethylene based polymers such as ethylene/vinyl acetate, ethylene acrylate, ethylene methacrylate, ethylene methylacrylate, ethylene methyl methacrylate, copolymers of ethylene and 1-6-mono or di-unsaturated monomers, polyamides, polybutadiene rubber, polyesters such as polyethylene therephthalate, polybutylene therephthalate, thermoplastic polycarbonates, atactic poly-alpha-olefins including atactic polyethylene, atactic polypropylene and others; thermoplastic polyacrylamides, polyacrylonitrile, copolymers

EP 0 410 412 B1

of acrylonitrile with other monomers such as butadiene, styrene etc., polymethyl pentene, polyphenylene sulfide, aromatic polyurethanes; styreneacrylonitrile, acrylonitrile-butadiene-styrene, styrenebutadiene rubbers, polyethylene therephthalate, acrylonitrile-butadiene-styrene elastomers and polyphenylene sulfide.

16. The composition of Claim 15, wherein the thermoplastic copolymer comprises an ethylene-vinyl acetate polymer.

17. The composition of Claim 16, wherein the composition has an initial peel strength immediately after application of at least 1.1 kg/cm (6 lb./inch) and final equilibrium peel strength of less than 0.18 kg/cm (1 lb./inch).

18. The composition of any one of Claims 13 to 17 comprising:
(a) 5 to 40 weight-% of a cyclohexane dimethanol dibenzoate plasticizer compound;
(b) 20 to 80 weight-% of a tackifier selected from the group consisting of an aromatic, aromatic-aliphatic, aliphatic or rosin tackifying resin and
(c) 0 to 15 weight-% of a thermoplastic ethylene vinyl acetate copolymer;
wherein the composition, after application, does not attain a final equilibrium bond peel strength for at least 30 minutes after application.

19. A case or carton, especially a cigarette carton secured by an effective amount of the hot melt adhesive according to any one of Claims 13 through 18.

20. A pallet comprising an assembly of cases or cartons secured by an effective amount of the hot melt adhesive according to any one of Claims 13 to 18.

21. A coupon or a label removably adhesively attached by means of a hot melt adhesive composition according to any one of Claims 13 through 18.

22. A disposable article comprising an absorbent layer adhered to a substrate, especially an outer wrap, by the adhesive according to any one of Claims 1 through 12.

23. The article of Claim 22, wherein the article is a feminine pad.

24. The article of Claim 22, wherein the article is a disposable diaper.

25. The article of any one of Claims 22 through 24, the composition being sprayed on the layer and/or the substrate.

**Patentansprüche**

**Patentansprüche für folgende Vertragsstaaten : AT, BE, CH, DE, FR, GB, IT, LI, LU, NL, SE**

1. Heißschmelzklebstoffzusammensetzung, umfassend:
(a) 5 bis 50 Gew. -% eines Weichmachers, wobei der Weichmacher bei Raumtemperatur ein Feststoff ist und eine Erweichungstemperatur von mindestens 45°C hat;
(b) 20 bis 80 Gew.-% eines Klebrigmachers, ausgewählt aus der Gruppe, die aus einem aromatischen, einem aromatisch-aliphatischen, einem aliphatischen oder einem natürlichen Klebrigmacherharz besteht; und
(c) 0 bis 55 Gew.-% eines thermoplastischen Polymers, ausschließlich thermoplastischer Blockcopolymere bestehend aus einem A-B-A-Blockcopolymer, einem A(B-A)$_n$-B-A-Blockcopolymer, worin n eine ganze Zahl von 1 oder mehr ist, oder einem radialen Blockcopolymer, wobei jedes A einen Polystyrolblock und jedes B einen gummiartigen Block umfaßt,
worin die Zusammensetzung nach ihrer Aufbringung und Abkühlung bezüglich einer physikalischen Eigenschaft, ausgewählt aus der Gruppe Lagermodul (G'), Ablösefestigkeit und Scherfestigkeit, mindestens fünf Minuten lang keinen Umgebungszustand einnimmt.

2. Zusammensetzung nach Anspruch 1, dadurch gekennzeichnet, daß der Weichmacher ein Diester ist, der

24

einen Diester einer cyclischen dihydroxy-substituierten aromatischen oder aliphatischen Verbindung und einer cyclischen aliphatischen oder aromatischen Mono-Carbonsäureverbindung; oder einen Diester einer cyclischen dicarbonsäure-substituierten aliphatischen oder aromatischen Verbindung und einer cyclischen hydroxy-substituierten aliphatischen oder aromatischen Verbindung umfaßt.

3. Zusammensetzung nach Anspruch 2, worin der Weichmacher einen aromatischen Carbonsäureester eines polyfunktionellen Alkohols mit 1 bis 10 Hydroxylgruppen, vorzugsweise einer Dihydroxycyclohexanverbindung und insbesondere einer 1,4-substituierten Cyclohexanverbindung umfaßt.

4. Zusammensetzung nach Anspruch 3, worin der Weichmacher eine Cyclohexandimethanoldibenzoatverbindung ist, insbesondere 1,4-Cyclohexandimethanoldibenzoat.

5. Zusammensetzung nach einem der Ansprüche 1 bis 4, worin der Weichmacher eine Mischung aus cis- und trans-Isomeren umfaßt.

6. Zusammensetzung nach einem der Ansprüche 1 bis 5, worin der Weichmacher eine Erweichungstemperatur von mindestens 60°C hat.

7. Zusammensetzung nach Anspruch 1, worin der Weichmacher einen cycloaliphatischen oder aromatischen Ester einer Benzoldicarbonsäure, vorzugsweise einer Benzol-1,4-dicarbonsäure und insbesondere Dicyclohexylorthophthalat oder Diphenylorthophthalat umfaßt.

8. Zusammensetzung nach Anspruch 1, worin der Weichmacher eine aromatische Sulfonamidverbindung entsprechend der allgemeinen Formel

$$R - Ar - SO_2 - NR_2$$

umfaßt, worin jedes R unabhängig aus der Gruppe ausgewählt ist, die aus H, aliphatischen und cycloaliphatischen Gruppen mit 1 bis 12 Kohlenstoffatomen besteht, wobei R vorzugsweise Methyl, Ethyl oder Cyclohexyl ist; und/oder das Formaldehydkondensationsprodukt derartiger Sulfonamidweichmacherverbindungen umfaßt.

9. Zusammensetzung nach einem der Ansprüche 1 bis 8, worin das thermoplastische Polymer aus Polymeren auf Ethylenbasis ausgewählt ist, z. B. Ethylen-Vinylacetat, Ethylenacrylat, Ethylenmethacrylat, Ethylenmethylacrylat, Ethylenmethylmethacrylat, Copolymere von Ethylen und 1-6-mono- oder di-ungesättigten Monomeren, Polyamide, Polybutadienkautschuk, Polyester wie Polyethylenterephthalat, Polybutylenterephthalat, thermoplastische Polycarbonate, ataktische Poly-$\alpha$-Olefine einschließlich ataktischem Polyethylen, ataktischem Polypropylen und anderen; thermoplastische Polyacrylamide, Polyacrylnitril, Copolymere von Acrylnitril mit anderen Monomeren wie Butadien, Styrol etc., Polymethylpenten, Polyphenylensulfid, aromatische Polyurethane; Styrolacrylnitril, Acrylnitrilbutadienstyrol, Styrolbutadienkautschuke, Polyethylenterephthalat, Acrylnitrilbutadienstyrolelastomere und Polyphenylensulfid.

10. Zusammensetzung nach einem der Ansprüche 1 bis 9, worin das thermoplastische Polymer in einer Konzentration von 5 bis 40 Gew.-% vorliegt.

11. Zusammensetzung nach Anspruch 9 oder 10, worin der Lagermodul des Klebstoffes mindestens nach fünf Minuten nach Aufbringung und Abkühlung der Zusammensetzung gegenüber dem Modul der Zusammensetzung bei deren erster Abkühlung mindestens 10fach höher ist.

12. Zusammensetzung nach Anspruch 9, 10 oder 11, umfassend:
(a) 5 bis 50 Gew.-% eines Cyclohexandimethanoldibenzoat-Weichmachers;
(b) 20 bis 80 Gew.-% eines Klebrigmachers, ausgewählt aus der Gruppe, die aus einem aromatischen, einem aromatisch-aliphatischen, einem aliphatischen oder einem natürlichen Klebrigmacherharz besteht; und
(c) 10 bis 55 Gew.-% eines thermoplastischen Blockcopolymers;
worin die Zusammensetzung nach ihrer Aufbringung in einer physikalischen Eigenschaft, ausgewählt aus der Gruppe Lagermodul (G'), Ablösefestigkeit oder Scherfestigkeit, 5 bis 10 Minuten nach Aufbringung bei Umgebungstemperaturen am Arbeitsplatz keinen endgültigen Gleichgewichtswert annimmt.

13. Zusammensetzung nach einem der Ansprüche 1 bis 9, umfassend
(a) 5 bis 50 Gew.-% eines Weichmachers, der einen Diester einer cyclischen dihydroxy-substituierten aromatischen oder aliphatischen Verbindung und einer cyclischen aliphatischen oder aromatischen Mono-Carbonsäureverbindung umfaßt; oder einen Diester einer cyclischen dicarbonsäure-substituierten aliphatischen oder aromatischen Verbindung und einer cyclischen hydroxy-substituierten aliphatischen oder aromatischen Verbindung, wobei der Weichmacher bei Raumtemperatur ein Feststoff ist und eine Erweichungstemperatur von mindestens 45°C hat;

(b) 20 bis 80 Gew. -% eines Klebrigmachers, ausgewählt aus der Gruppe, die aus einem aromatischen, einem aromatisch-aliphatischen, einem aliphatischen oder einem natürlichen Klebrigmacherharz besteht; und

(c) fakultativ bis zu 15 Gew.-% eines thermoplastischen Polymers;

wobei die Heißschmelzzusammensetzung so formuliert ist, daß sie bei ihrer Aufbringung eine flüchtige Kohäsivbindung bildet, welche anfangs unter Scher- und Ablösebelastung fest ist, sich jedoch mit der Zeit zu einer spröden Bindung mit erheblicher Scherfestigkeit, aber nur geringer Ablösefestigkeit verändert.

14. Zusammensetzung nach Anspruch 13, worin das thermoplastische Copolymer ein Ethylen-Vinylacetat-Polymer umfaßt.

15. Zusammensetzung nach Anspruch 14, worin die Zusammensetzung unmittelbar nach ihrer Aufbringung eine anfängliche Ablösefestigkeit von mindestens 1,1 kg/cm (6 lb. /inch sowie eine Endgleichgewichts-Ablösefestigkeit von weniger als 0,18 kg/cm (1 lb./inch) hat.

16. Zusammensetzung nach einem der Ansprüche 13 bis 15, umfassend :

(a) 5 bis 40 Gew. -% einer Cyclohexandimethanoldibenzoat-Weichmacherverbindung;

(b) 20 bis 80 Gew. -% eines Klebrigmachers, ausgewählt aus der Gruppe, die aus einem aromatischen, einem aromatisch-aliphatischen, einem aliphatischen oder einem natürlichen Klebrigmacherharz besteht; und

(c) 0 bis 15 Gew. -% eines thermoplastischen Ethylen-Vinylacetat-Copolymers;

worin die Zusammensetzung nach ihrer Aufbringung mindestens 30 Minuten nach Aufbringung keine Endgleichgewichts-Ablösefestigkeit hat.

17. Schachtel oder Karton, insbesondere Zigarettenkarton, der mit einer wirkungsvollen Menge des Heißschmelzklebstoffes gemäß einem der Ansprüche 13 bis 16 geklebt ist.

18. Palette, umfassend eine Anordnung von Schachteln oder Kartons, die mit einer wirkungsvollen Menge des Heißschmelzklebstoffes gemäß einem der Ansprüche 13 bis 16 geklebt sind.

19. Coupon oder Etikett, das mittels der Heißschmelzkleberzusammensetzung gemäß einem der Ansprüche 13 bis 16 lösbar aufgeklebt ist.

20. Wegwerfartikel, umfassend eine mittels des Klebstoffes gemäß einem der Ansprüche 1 bis 12 auf ein Substrat, insbesondere eine äußere Umhüllung, aufgeklebte Absorptionsschicht.

21. Artikel nach Anspruch 20, worin der Artikel eine Damenslipeinlage ist.

22. Artikel nach Anspruch 20, worin der Artikel eine Wegwerfwindel ist.

23. Artikel nach einem der Ansprüche 20 bis 2, worin die Zusammensetzung auf die Schicht und/oder das Substrat aufgesprüht ist.

**Patentansprüche für folgende Vertragsstaaten : DK, ES, GR**

1. Heißschmelzklebstoffzusammensetzung, umfassend:

(a) 5 bis 50 Gew.-% eines Weichmachers, wobei der Weichmacher bei Raumtemperatur ein Feststoff ist und eine Erweichungstemperatur von mindestens 45°C hat;

(b) 20 bis 80 Gew. -% eines Klebrigmachers, ausgewählt aus der Gruppe, die aus einem aromatischen, einem aromatisch-aliphatischen, einem aliphatischen oder einem natürlichen Klebrigmacherharz besteht; und

(c) 0 bis 55 Gew.-% eines thermoplastischen Polymers,

worin die Zusammensetzung nach ihrer Aufbringung und Abkühlung bezüglich einer physikalischen Eigenschaft, ausgewählt aus der Gruppe Lagermodul (G'), Ablösefestigkeit und Scherfestigkeit, minde-

EP 0 410 412 B1

stens fünf Minuten lang keinen Umgebungszustand einnimmt.

2. Zusammensetzung nach Anspruch 1, dadurch gekennzeichnet, daß der Weichmacher ein Diester ist, der einen Diester einer cyclischen dihydroxy-substituierten aromatischen oder aliphatischen Verbindung und einer cyclischen aliphatischen oder aromatischen Mono-Carbonsäureverbindung; oder einen Diester einer cyclischen dicarbonsäure-substituierten aliphatischen oder aromatischen Verbindung und einer cyclischen hydroxy-substituierten aliphatischen oder aromatischen Verbindung umfaßt.

3. Zusammensetzung nach Anspruch 2, worin der Weichmacher einen aromatischen Carbonsäureester eines polyfunktionellen Alkohols mit 1 bis 10 Hydroxylgruppen, vorzugsweise einer Dihydroxycyclohexanverbindung und insbesondere einer 1,4-substituierten Cyclohexanverbindung umfaßt.

4. Zusammensetzung nach Anspruch 3, worin der Weichmacher eine Cyclohexandimethanoldibenzoatverbindung ist, insbesondere 1,4-Cyclohexandimethanoldibenzoat.

5. Zusammensetzung nach einem der Ansprüche 1 bis 4, worin der Weichmacher eine Mischung aus cis- und trans-Isomeren umfaßt.

6. Zusammensetzung nach einem der Ansprüche 1 bis 5, worin der Weichmacher eine Erweichungstemperatur von mindestens 60°C hat.

7. Zusammensetzung nach Anspruch 1, worin der Weichmacher einen cycloaliphatischen oder aromatischen Ester einer Benzoldicarbonsäure, vorzugsweise einer Benzol-1,4-dicarbonsäure und insbesondere Dicyclohexylorthophthalat oder Diphenylorthophthalat umfaßt.

8. Zusammensetzung nach Anspruch 1, worin der Weichmacher eine aromatische Sulfonamidverbindung entsprechend der allgemeinen Formel

$$R - Ar - SO_2 - NR_2$$

umfaßt, worin jedes R unabhängig aus der Gruppe ausgewählt ist, die aus H, aliphatischen und cycloaliphatischen Gruppen mit 1 bis 12 Kohlenstoffatomen besteht, wobei R vorzugsweise Methyl, Ethyl oder Cyclohexyl ist; und/oder das Formaldehydkondensationsprodukt derartiger Sulfonamidweichmacherverbindungen umfaßt.

9. Zusammensetzung nach einem der Ansprüche 1 bis 8, worin das thermoplastische Polymer in einer Konzentration von 5 bis 40 Gew.-% vorliegt.

10. Zusammensetzung nach Anspruch 9, worin das thermoplastische Polymer ein Blockcopolymer, vorzugsweise ein A-B-A-Blockcopolymer, umfaßt, worin A einen Styrolblock und B einen gummiartigen Block umfaßt.

11. Zusammensetzung nach Anspruch 9 oder 10, worin der Lagermodul des Klebstoffes mindestens nach fünf Minuten nach Aufbringung und Abkühlung der Zusammensetzung gegenüber dem Modul der Zusammensetzung bei deren erster Abkühlung mindestens 10fach höher ist.

12. Zusammensetzung nach Anspruch 10 oder 11, umfassend:
(a) 5 bis 50 Gew.-% eines Cyclohexandimethanoldibenzoat-Weichmachers;
(b) 20 bis 80 Gew.-% eines Klebrigmachers, ausgewählt aus der Gruppe, die aus einem aromatischen, einem aromatisch-aliphatischen, einem aliphatischen oder einem natürlichen Klebrigmacherharz besteht; und
(c) 10 bis 55 Gew.-% eines thermoplastischen Blockcopolymers, insbesondere eines A-B-A-Blockcopolymers, in dem jedes A einen Styrolblock und jedes B ein gummiartiges Blockcopolymer umfaßt;
worin die Zusammensetzung nach ihrer Aufbringung bezüglich einer physikalischen Eigenschaft, ausgewählt aus der Gruppe Lagermodul (G'), Ablösefestigkeit oder Scherfestigkeit, 5 bis 10 Minuten nach Aufbringung bei Umgebungstemperaturen am Arbeitsplatz keinen endgültigen Gleichgewichtswert annimmt.

13. Zusammensetzung nach einem der Ansprüche 1 bis 9, umfassend
(a) 5 bis 50 Gew.-% eines Weichmachers, wobei der Weichmacher bei Raumtemperatur ein Feststoff ist und eine Erweichungstemperatur von mindestens 45°C hat;
(b) 20 bis 80 Gew.-% eines Klebrigmachers, ausgewählt aus der Gruppe, die aus einem aromatischen, einem aromatisch-aliphatischen, einem aliphatischen oder einem natürlichen Klebrigmacherharz be-

27

steht; und

(c) fakultativ bis zu 15 Gew.-% eines thermoplastischen Polymers;

wobei die Heißschmelzzusammensetzung so formuliert ist, daß sie bei ihrer Aufbringung eine flüchtige Kohäsivbindung bildet, welche anfangs unter Scher- und Ablösebelastung fest ist, sich jedoch mit der Zeit zu einer spröden Bindung mit erheblicher Scherfestigkeit, aber nur geringer Ablösefestigkeit verändert.

14. Zusammensetzung nach Anspruch 13, worin das thermoplastische Copolymer in einer Konzentration von 0,1 bis 10 Gew.-% vorliegt.

15. Zusammensetzung nach einem der Ansprüche 1 bis 8, worin das thermoplastische Polymer aus Polymeren auf Ethylenbasis ausgewählt ist, z. B. Ethylen-Vinylacetat, Ethylenacrylat, Ethylenmethacrylat, Ethylenmethylacrylat, Ethylenmethylmethacrylat, Copolymere von Ethylen und 1-6-mono- oder di-ungesättigten Monomeren, Polyamide, Polybutadienkautschuk, Polyester wie Polyethylenterephthalat, Polybutylenterephthalat, thermoplastische Polycarbonate, ataktische Poly-α-Olefine einschließlich ataktischem Polyethylen, ataktischem Polypropylen und anderen; thermoplastische Polyacrylamide, Polyacrylnitril, Copolymere von Acrylnitril mit anderen Monomeren wie Butadien, Styrol etc., Polymethylpenten, Polyphenylensulfid, aromatische Polyurethane; Styrolacrylnitril, Acrylnitrilbutadienstyrol, Styrolbutadienkautschuke, Polyethylenterephthalat, Acrylnitrilbutadienstyrolelastomere und Polyphenylensulfid.

16. Zusammensetzung nach Anspruch 15, worin das thermoplastische Copolymer ein Ethylen-Vinylacetat-Polymer umfaßt.

17. Zusammensetzung nach Anspruch 16, worin die Zusammensetzung unmittelbar vor ihrer Aufbringung eine anfängliche Ablösefestigkeit von mindestens 1,1 kg/cm (6 lb./inch) und eine Endgleichgewichts-Ablösefestigkeit von weniger als 0,18 kg/cm (1 lb./inch) hat.

18. Zusammensetzung nach einem der Ansprüche 13 bis 17, umfassend :

(a) 5 bis 40 Gew.-% einer Cyclohexandimethanoldibenzoat-Weichmacherverbindung;

(b) 20 bis 80 Gew.-% eines Klebrigmachers, ausgewählt aus der Gruppe, die aus einem aromatischen, einem aromatisch-aliphatischen, einem aliphatischen oder einem natürlichen Klebrigmacherharz besteht; und

(c) 0 bis 15 Gew.-% eines thermoplastischen Ethylen-Vinylacetat-Copolymers;

worin die Zusammensetzung nach ihrer Aufbringung mindestens 30 Minuten nach Aufbringung keine Endgleichgewichts-Ablösefestigkeit hat.

19. Schachtel oder Karton, insbesondere Zigarettenkarton, der mit einer wirkungsvollen Menge des Heißschmelzklebstoffes gemäß einem der Ansprüche 13 bis 18 geklebt ist.

20. Palette, umfassend eine Anordnung von Schachteln oder Kartons, die mit einer wirkungsvollen Menge des Heißschmelzklebstoffes gemäß einem der Ansprüche 13 bis 18 geklebt sind.

21. Coupon oder Etikett, das mittels der Heißschmelzkleberzusammensetzung gemäß einem der Ansprüche 13 bis 18 lösbar aufgeklebt ist.

22. Wegwerfartikel, umfassend eine mittels des Klebstoffes gemäß einem der Ansprüche 1 bis 12 auf ein Substrat, insbesondere eine äußere Umhüllung, aufgeklebte Absorptionsschicht.

23. Artikel nach Anspruch 22, worin der Artikel eine Damenslipeinlage ist.

24. Artikel nach Anspruch 22, worin der Artikel eine Wegwerfwindel ist.

25. Artikel nach einem der Ansprüche 22 bis 24, worin die Zusammensetzung auf die Schicht und/oder das Substrat aufgesprüht ist.

## Revendications

**Revendications pour les Etats contractants suivants : AT, BE, CH, DE, FR, GB, IT, LI, LU, NL, SE**

1. Une composition adhésive thermofusible comprenant :
   (a) de 5 à 50 % en poids d'un plastifiant, ledit plastifiant étant un solide à la température ambiante et ayant un point de ramollissement d'au moins 45°C,
   (b) de 20 à 80 % en poids d'un agent de collage choisi dans le groupe consistant en une résine collante aromatique, aromatique-aliphatique, aliphatique ou de rosine, et
   (c) de 0 à 55 % en poids d'un polymère thermoplastique excluant les copolymères en bloc thermoplastiques consistant en un copolymère en bloc A-B-A,
   un copolymère en bloc A-(B-A)$_n$-B-A, dans lequel n est un entier de 1 ou plus, ou un copolymère en bloc radial, chaque A comprenant un bloc polystyrène et chaque B comprenant un bloc caoutchouteux,
   dans laquelle la composition, après application et refroidissement, n'atteint pas un état ambiant pour une propriété physique choisie dans le groupe consistant en : le module de conservation (G'), la résistance à l'écaillement, et la résistance au cisaillement pendant au moins cinq minutes.

2. La composition de la revendication 1, ledit plastifiant étant un diester qui comprend un diester d'un composé cyclique aliphatique ou aromatique substitué par deux hydroxy et d'un composé acide monocarboxylique aromatique ou aliphatique cyclique ; ou un diester d'un composé aromatique ou aliphatique cyclique substitué par un acide dicarboxylique et d'un composé aromatique ou aliphatique cyclique substitué par un hydroxy.

3. La composition de la revendication 2, dans laquelle le plastifiant comprend un ester acide carboxylique aromatique d'un alcool polyfonctionnel ayant de 1 à 10 groupes hydroxy, de préférence d'un composé cyclohexane dihydroxy et particulièrement d'un composé cyclohexane 1,4-substitué.

4. La composition de la revendication 3, dans laquelle le plastifiant est un composé dibenzoate diméthanol cyclohexane, particulièrement un dibenzoate diméthanol 1,4-cyclohexane.

5. La composition selon l'une quelconque des revendications 1 à 4, dans laquelle le plastifiant comprend un mélange d'isomères cis et trans.

6. La composition selon l'une quelconque des revendications 1 à 5, dans laquelle le plastifiant a un point de ramollissement d'au moins 60° C.

7. La composition de la revendication 1, dans laquelle le plastifiant comprend un ester cycloaliphatique ou aromatique d'un acide dicarboxylique benzène, de préférence un acide benzène-1,4-dicarboxylique et particulièrement un orthophtalate de dicyclohexyle ou un orthophtalate de diphényle.

8. La composition de la revendication 1, dans laquelle le plastifiant comprend un composé sulfonamide aromatique, correspondant à la formule générale :
   $$R\text{-}A_r\text{-}So_2\text{-}NR_2,$$
   dans laquelle chaque R est choisi indépendamment dans le groupe consistant en -H, les radicaux aliphatiques et cycloaliphatiques ayant de 1 à 12 atomes de carbone, R étant de préférence méthyle, éthyle ou cyclohexyle ; et/ou comprend le produit de condensation formaldéhyde de tels composés plastifiants sulfonamide.

9. La composition selon l'une quelconque des revendications 1 à 8, dans laquelle le polymère thermoplastique est choisi à partir de polymères à base d'éthylène tels que l'acétate d'éthylène/vinyle, l'acrylate d'éthylène, le méthacrylate d'éthylène, le méthylacrylate d'éthylène, le méthacrylate d'éthylène méthyle, les copolyméres d'éthylène et de monomères 1-6 mono ou di-insaturés, les polyamides, le caoutchouc de polybutadiène, les polyesters tels que le théréphtalate de polybutylène, les polycarbonates thermoplastiques, les poly-alpha-oléfines atactiques incluant le polyéthylène atactique, le polypropylène atactique et autres ; les polyacrylamides thermoplastiques, le polyacrylonitrile, les copolymères d'acrylonitrile avec d'autres monomères tels que le butadiène, le styrène, etc ..., le pentène polyméthyle, le sulfure de polyphénylène, les polyuréthanes aromatiques l'acrylonitrile-styrène, l'acrylonitrile-butadiènestyrène, les caoutchoucs de styrène-butadiène, le téréphtalate de polyéthylène, les élastomères d'acrylonitrile-butadiène-styrène et le sulfure de polyphénylène.

10. La composition selon l'une des revendications 1 à 9, dans laquelle le polymère thermoplastique est présent à une concentration de 5 à 40 % en poids.

11. La composition de la revendication 9 ou 10, dans laquelle au moins cinq minutes après que la composition soit appliquée et refroidie, l'adhésif montre une augmentation d'au moins dix fois du module de conservation par rapport au module de la composition quand elle est refroidie en premier.

12. La composition de la revendication 9, 10 ou 11 comprenant :
    (a) de 5 à 50 % en poids d'un plastifiant dibenzoate diméthanol cyclohexane,
    (b) de 20 à 80 % en poids d'un agent de collage choisi dans le groupe consistant en une résine collante aromatique, aromatique-aliphatique, aliphatique ou de rosine, et
    (c) de 10 à 55 % en poids d'un copolymère en bloc thermoplastique ;
    dans laquelle la composition, après application, n'atteint pas une valeur d'équilibre finale pour une propriété physique choisie dans le groupe de propriétés consistant en : le module de conservation (G'), la résistance à l'écaillement ou la résistance au cisaillement pendant cinq à dix minutes après application aux températures ambiantes à la place d'utilisation.

13. La composition selon l'une quelconque des revendications 1 à 9 comprenant :
    (a) de 5 à 50 % en poids d'un plastifiant qui comprend un diester d'un composé aromatique ou aliphatique cyclique substitué par deux hydroxy et un composé acide mono-carboxylique aromatique ou aliphatique cyclique ; ou un diester d'un composé aromatique ou aliphatique cyclique substitué par un acide dicarboxylique et d'un composé aliphatique ou aromatique cyclique substitué par un hydroxy, ledit plastifiant étant un solide à la température ambiante et ayant un point de ramollissement d'au moins 45° C;
    (b) de 20 à 80 % en poids d'un agent de collage choisi dans le groupe consistant en une résine collante aromatique, aromatique-aliphatique, aliphatique ou de rosine ; et
    (c) éventuellement, jusqu'à 15 % en poids d'un polymère thermoplastique ; ladite composition thermofusible étant formulée pour former, après application, une liaison cohésive éphémère qui est initialement forte au cisaillement et à l'écaillement, ladite liaison changeant avec le temps en une liaison fragile qui a une résistance au cisaillement importante mais une faible résistance à l'écaillement.

14. La composition de la revendication 13, dans laquelle le copolymère thermoplastique comprend un polymère d'acétate d'éthylène-vinyle.

15. La composition de la revendication 14, dans laquelle la composition a une résistance à l'écaillement initiale immédiatement après application d'au moins 1,1 Kg/cm (6lb./inch) et une résistance à l'écaillement à l'équilibre finale inférieure à 0,18 Kg/cm (1 lb./inch).

16. La composition selon l'une quelconque des revendications 13 à 15, comprenant :
    (a) de 5 à 40 % en poids d'un composé plastifiant dibenzoate diméthanol cyclohexane ;
    (b) de 20 à 80 % en poids d'un agent de collage choisi dans le groupe consistant en une résine collante aromatique, aromatique-aliphatique, aliphatique ou de rosine ; et
    (c) de 0 à 15 % en poids d'un copolymère d'acétate de vinyle éthylène thermoplastique ; dans laquelle la composition, après application, n'atteint pas une résistance à l'écaillement de la liaison à l'équilibre finale pendant au moins trente minutes après application.

17. Une boîte ou un carton, particulièrement un carton pour cigarettes fixé par une quantité suffisante de l'adhésif thermofusible selon l'une quelconque des revendications 13 à 16.

18. Une palette comprenant un assemblage de boîtes ou cartons fixés par une quantité suffisante de l'adhésif thermofusible selon l'une quelconque des revendications 13 à 16.

19. Un ticket ou une étiquette attachée de façon adhésive et détachable au moyen d'une composition adhésive thermofusible selon l'une des revendications 13 à 16.

20. Un article jetable comprenant une épaisseur absorbante fixée à un substrat, en particulier à une enveloppe extérieure, par l'adhésif selon l'une quelconque des revendications 1 à 12.

**21.** L'article de la revendication 20, dans lequel l'article est une serviette hygiénique.

**22.** L'article de la revendication 20, dans lequel l'article est une couche jetable.

**23.** L'article selon l'une quelconque des revendications 20 à 22, la composition étant vaporisée sur l'épaisseur et/ou le substrat.

**Revendications pour les Etats contractants suivants : DK, ES, GR**

**1.** Une composition adhésive thermofusible comprenant :
(a) de 5 à 50 % en poids d'un plastifiant, ledit plastifiant étant un solide à la temperature ambiante et ayant un point de ramollissement d'au moins 45°C,
(b) de 20 à 80 % en poids d'un agent de collage choisi dans le groupe consistant en une résine collante aromatique, aromatique-aliphatique, aliphatique ou de rosine, et
(c) de 0 à 55 % en poids d'un polymère thermoplastique excluant les copolymères en bloc thermoplastiques consistant en un copolymère en bloc A-B-A,
dans laquelle la composition, après application et refroidissement, n'atteint pas un état ambiant pour une propriété physique choisie dans le groupe consistant en : le module de conservation (G'), la résistance à l'écaillement, et la résistance au cisaillement pendant au moins cinq minutes.

**2.** La composition de la revendication 1, ledit plastifiant étant un diester qui comprend un diester d'un composé cyclique aliphatique ou aromatique substitué par deux hydroxy et d'un composé acide monocarboxylique aromatique ou aliphatique cyclique ; ou un diester d'un composé aromatique ou aliphatique cyclique substitué par un acide dicarboxylique et d'un composé aromatique ou aliphatique cyclique substitué par un hydroxy.

**3.** La composition de la revendication 2, dans laquelle le plastifiant comprend un ester acide carboxylique aromatique d'un alcool polyfonctionnel ayant de 1 à 10 groupes hydroxy, de préférence d'un composé cyclohexane dihydroxy et particulièrement d'un composé cyclohexane 1,4-substitué.

**4.** La composition de la revendication 3, dans laquelle le plastifiant est un composé dibenzoate diméthanol cyclohexane, particulièrement un dibenzoate 1,4-cyclohexane.

**5.** La composition selon l'une quelconque des revendications 1 à 4, dans laquelle le plastifiant comprend un mélange d'isomères cis et trans.

**6.** La composition selon l'une quelconque des revendications 1 à 5, dans laquelle le plastifiant a un point de ramollissement d'au moins 60° C.

**7.** La composition de la revendication 1, dans laquelle le plastifiant comprend un ester cycloaliphatique ou aromatique d'un acide dicarboxylique benzène, de préférence un acide benzène-1,4-dicarboxylique et particulièrement un orthophtalate de dicyclohexyle ou un orthophtalate de diphényle.

**8.** La composition de la revendication 1, dans laquelle le plastifiant comprend un composé sulfonamide aromatique, correspondant à la formule générale :
$$R\text{-}A_r\text{-}So_2\text{-}NR_2,$$
dans laquelle chaque R est choisi indépendamment dans le groupe consistant en -H, les radicaux aliphatiques et cycloaliphatiques ayant de 1 à 12 atomes de carbone, R étant de préférence méthyle, éthyle ou cyclohexyle ; et/ou comprend le produit de condensation formaldéhyde de tels composés plastifiants sulfonamide.

**9.** La composition selon l'une quelconque des revendications 1 à 8, dans laquelle le polymère thermoplastique est présent à une concentration de 5 à 40 % en poids.

**10.** La composition de la revendication 9, dans laquelle le polymère thermoplastique comprend un copolymère en bloc, de préférence un copolymère en bloc A-B-A dans lequel A comprend un bloc styrène et B comprend un bloc caoutchouteux.

**11.** La composition de la revendication 9 ou 10, dans laquelle au moins cinq minutes après que la composition soit appliquée et refroidie, l'adhésif montre une augmentation d'au moins dix fois du module de conservation par rapport au module de la composition quand elle est refroidie en premier.

**12.** La composition de la renvendication 10 ou 11 comprenant :

(a) de 5 à 50 % en poids d'un plastifiant dibenzoate diméthanol cyclohexane,

(b) de 20 à 80 % en poids d'un agent de collage choisi dans le groupe consistant en une résine collante aromatique, aromatique-aliphatique, aliphatique ou de rosine, et

(c) de 10 à 55 % en poids d'un copolymère en bloc thermoplastique ; particulièrement un copolymère en bloc A-B-A dans lequel chaque A comprend un bloc styrène et chaque B comprend un copolymère en bloc caoutchouteux ;

dans lesquelles la composition, après application, n'atteint pas une valeur d'équilibre finale pour une propriété physique choisie dans le groupe de propriétés consistant en : le module de conservation (G'), la résistance à l'écaillement ou la résistance au cisaillement pendant cinq à dix minutes après application aux températures ambiantes à la place d'utilisation.

**13.** La composition selon l'une quelconque des revendications 1 à 9 comprenant :

(a) de 5 à 50 % en poids d'un plastifiant, ledit plastifiant étant un solide à la température ambiante et ayant un point de ramollissement d'au moins 45° C ;

(b) de 20à 80 % en poids d'un agent de collage choisi dans le groupe consistant en une résine collante aromatique, aromatique-aliphatique, aliphatique ou de rosine ; et

(c) éventuellement, jusqu'à 15 % en poids d'un polymère thermoplastique ; ladite composition thermofusible étant formulée pour former, après application, une liaison cohésive éphémère qui est initialement forte au cisaillement et à l'écaillement, ladite liaison changeant avec le temps en une liaison fragile qui a une résistance au cisaillement importante mias une faible résistance à l'écaillement.

**14.** La composition de la revendication 13, dans laquelle le polymère thermoplastique est présent à une concentration de 0,1 à 10 % en poids.

**15.** La composition selon l'une quelconque des revendications 1 à 8, dans laquelle le polymère thermoplastique est choisi à partir de polymères à base d'éthylène tels que l'acétate d'éthylène/vinyle, l'acrylate d'éthylène, le méthacrylate d'éthylène, le méthylacrylate d'éthylène, le méthacrylate d'éthylène méthyle, les copolymères d'éthylène et de monomères 1-6 mono ou di-insaturés, les polyamides, le caoutchouc de polybutadiène, les polyesters tels que le théréphtalate de polybutylène, les polycarbonates thermoplastiques, les poly-alpha-oléfines atactiques incluant le polyéthylène atactique, le polypropylène atactique et autres, les polyacrylamides thermoplastiques, le polyacrylonitrile, les copolymères d'acrylonitrile avec d'autres monomères tels que le butadiène, le styrène, etc ..., le pentène polyméthyle, le sulfure de polyphénylène, les polyuréthanes aromatiques l'acrylonitrile-styrène, l'acrylonitrile-butadiènestyrène, les caoutchoucs de styrène-butadiène, le téréphtalate de polyéthylène, les élastomères d'acrylonitrile-butadiène-styrène et le sulfure de polyphénylène.

**16.** La composition de la revendication 15, dans laquelle le copolymère thermoplastique comprend un polymère d'acétate d'éthylène-vinyle.

**17.** La composition de la revendication 16, dans laquelle la composition a une résistance à l'écaillement initiale immédiatement après application d'au moins 1,1 Kg/cm (61b./inch) et une résistance à l'écaillement à l'équilibre finale inférieure à 0,18 Kg/cm (1 lb./inch).

**18.** La composition selon l'une quelconque des revendications 13 à 17, comprenant :

(a) de 5 à 40 % en poids d'un composé plastifiant dibenzoate diméthanol cyclohexane ;

(b) de 20 à 80 % en poids d'un agent de collage choisi dans le groupe consistant en une résine collante aromatique, aromatique-aliphatique, aliphatique ou de rosine ; et

(c) de 0 à 15 % en poids d'un copolymère d'acétate de vinyle éthylène thermoplastique ; dans laquelle la composition, après application, n'atteint pas une résistance à l'écaillement de la liaison à l'équilibre finale pendant au moins trente minutes après application.

**19.** Une boîte ou un carton, particulièrement un carton pour cigarettes fixé par une quantité suffisante de l'adhésif thermofusible selon l'une quelconque des revendications 13 à 18.

**20.** Une palette comprenant un assemblage de boîtes ou cartons fixés par une quantité suffisante de l'adhésif thermofusible selon l'une quelconque des revendications 13 à 18.

21. Un ticket ou une étiquette attachée de façon adhésive et détachable au moyen d'une composition adhésive thermofusible selon l'une des revendications 13 à 18.

22. Un article jetable comprenant une épaisseur absorbante fixée à un substrat, en particulier à une enveloppe extérieure, par l'adhésif selon l'une quelconque des revendications 1 à 12.

23. L'article de la revendication 22, dans lequel l'article est une serviette hygiénique.

24. L'article de la revendication 22, dans lequel l'article est une couche jetable.

25. L'article selon l'une quelconque des revendications 22 à 24, la composition étant vaporisée sur l'épaisseur et/ou le substrat.

FIG 1.

FIG 2

F163

DSC

MCAL/SEC

TEMPERATURE (C)

EP 0 410 412 B1

EP 0 410 412 B1

FIG 4

DSC

TEMPERATURE (C)

MCAL/SEC

37

Fig 5

DSC

TEMPERATURE (C)

MCAL/SEC

Fig 7

DSC

TEMPERATURE (C)

MCAL/SEC

F168

EP 0 410 412 B1

41

Fig 9

EP 0 410 412 B1

DSC

Fig 10

EP 0 410 412 B1

Fig 12

DSC

TEMPERATURE (C)

MCAL/SEC

Fig 13

EP 0 410 412 B1

46

DSC

FIG14

EP 0 410 412 B1

Fig 15

MCAL/SEC

1.00

0.50

0.00

-30.00   -10.00   10.00   30.00   50.00   70.00   90.00   110.00   130.00

TEMPERATURE (C)

DSC

EP 0 410 412 B1

48

Fig 9

DSC

TEMPERATURE (C)

MCAL/SEC

Fig 17

EP 0 410 412 B1

Fig 18

DSC

TEMPERATURE (C)

MCAL/SEC

Fig. 19

DSC

TEMPERATURE (C)

MCAL/SEC

Fig 20

Fig. 21

Fig. 22